(19) Europäisches Patentamt
European Patent Office
Office européen des brevets

(11) Publication number: **0 031 041 B1**

(12) **EUROPEAN PATENT SPECIFICATION**

(45) Date of publication of patent specification: **13.02.85**

(51) Int. Cl.$^4$: **C 07 C 69/743**, C 07 C 121/75, A 01 N 53/00, C 07 C 67/00

(21) Application number: **80107327.1**

(22) Date of filing: **25.11.80**

# (54) Pyrethroids and their preparation, their pesticidal composition; intermediates and their preparation.

(30) Priority: **26.11.79 IT 2754279**
**05.06.80 IT 2256680**

(43) Date of publication of application:
**01.07.81 Bulletin 81/26**

(45) Publication of the grant of the patent:
**13.02.85 Bulletin 85/07**

(84) Designated Contracting States:
**BE CH DE FR GB LI NL**

(56) References cited:
**BE-A- 858 137**
**FR-A-1 453 991**
**FR-A-2 281 918**
**FR-A-2 354 310**
**FR-A-2 381 033**

**CHEMICAL ABSTRACTS, vol. 86, no. 25, 20-06-1977, page 556, no. 189302q Columbus, Ohio, US**

(73) Proprietor: **Montedison S.p.A.**
**Patents & Licensing Dept. Foro Buonaparte, 31**
**P.O. Box 10528**
**I-20121 Milan (IT)**

(72) Inventor: **Bosone, Enrico, Dr.**
**1, Via Modestino**
**Milano (IT)**
Inventor: **Caprara, Giuseppe**
**17/1, Via Tolentino**
**Milano (IT)**
Inventor: **Corda, Francesco**
**13, Via Teodosio**
**Milano (IT)**
Inventor: **Gozzo, Franco, Dr.**
**52, Via Triulziana**
**San Donato Milanese (Milano) (IT)**
Inventor: **Menconi, Augusto, Dr.**
**Via Dogali 15**
**Crema (IT)**
Inventor: **Piccardi, Paolo**
**8, Via E. De Marchi**
**Milano (IT)**
Inventor: **Caprioli, Vincenzo**
**8, Via Loriga**
**San Martino (Pavia) (IT)**

**EP 0 031 041 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 69, no. 10, September 2, 1968 abstract 43254c Columbus, Ohio, US G. MAVEL et al.: "N.M.R. study of organophosphorus compounds. XIV. Conformation of substituted allylphosphonates"

CHEMICAL ABSTRACTS, vol. 59, no. 1, July 8, 1963, abstract 656d Columbus, Ohio, US B.I. IONIN et al.: "Prototropic isomerization of esters of alkenylphosphonic acids"

(74) Representative: Schmied-Kowarzik, Volker, Dr. et al
Patentanwälte Dr. V. Schmied-Kowarzik Dipl.-Ing. G. Dannenberg Dr. P. Weinhold Dr. D. Gudel Dipl.-Ing. S. Schubert Dr. P. Barz
Siegfriedstrasse 8
D-8000 München 40 (DE)

**Description**

The present invention concerns new insecticides and acaricides belonging to the class of pyrethroids, and more particularly, it relates to a new cyclopropanecarboxylic esters, substituted in position 3 by a dienic chain having from 4 to 8 carbon atoms and which is halosubstituted, the use of these compounds as insecticides and acaricides, as well as the intermediates and synthesis processes.

There are already known numerous pyrethroid insecticides, some of which combine a good insecticide activity with a sufficient persistence on the field (see for instance "Synthetic Pyrethroids" by M. Elliot — Editors ACS Simposium, Series No. 42, Washington 1977).

Likewise, there are known pyrethroids substituted in position 3 of the cyclopropylic ring by a dienic chain.

M. Elliot and collaborators described 5-benzyl-3-furylmethyl- esters of 2,2-dimethyl-3-butadienyl-cyclopropane-carboxylic acid [Nature No. *244*, p. 456 (1973)].

These compounds, endowed with a high insecticide activity, undergo, however, a fast degradation in the presence of air and light (photooxidation), wherefore they are not suited for use in the agriculture.

In Japanese Patent Appln. No. 7411854 by Sumitomo [Chemical Abstract No. *81*, 34599 (1974)] there are described esters with lower alcohols of 2,2-dimethyl-3-cyclopropanecarboxylic acids substituted in position 3 by a branched dienic chain having from 4 to 8 carbon atoms, and not substituted by halogens.

In Belgian Patent No. 858,137 (Bayer) there is described a method for the synthesis of intermediates for pyrethroids, among which also esters of 2-(β,δ,δ-trichloro-1,3-dienyl)-2,2-dimethyl-cyclopropanecarboxylic acid.

However, neither of this acid nor of its derivatives there have been given examples of their synthesis, nor there have been indicated the insecticide properties or its characteristics.

From the process, described in said patent, there can be deduced that the above mentioned carboxylic acid should be prpepared starting from 1,1,3-trichloro-6-methyl-heptatriene. No indications are given regarding the preparation of this compound or of other trienes, nor is there said anything about their use in the synthesis of pyrethroids.

Object of this invention are compounds of the general formula:

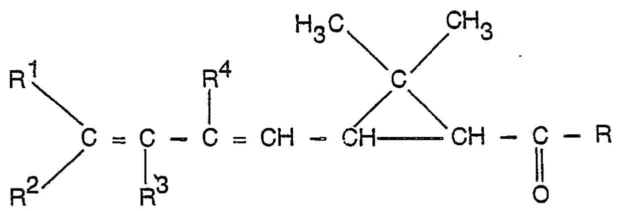

(I)

wherein: R = OH, O-alkyl $C_1$—$C_4$, halogen,

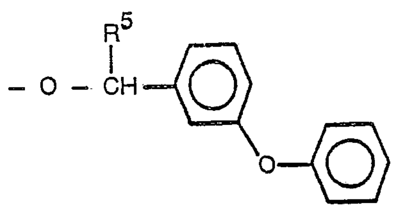

(wherein $R^5$ = H, CN, —C≡CH), $R^1$ = F, Cl, Br, $CH_3$, $CF_3$, $R^2$ = F, Cl, Br, $CF_3$, $R^3$ = H, F, Cl, Br, $CF_3$, $R^4$ = H, F, Cl, Br, $CF_3$; or $R^2$ and $R^3$ together form a third bond between the two carbon atoms to which they are bonded.

The compounds of general formula I, in which:

R =

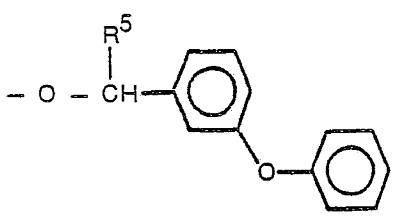

are insecticides and acaricides endowed with a high activity and which possess a high persistance of the action.

The compounds of general formula I, wherein R = OH, O-alkyl, halogen, are intermediates for the synthesis of the insecticide compounds.

In the above formulae alkyl $C_1$—$C_4$ is e.g. methyl, ethyl, propyl, i-propyl, butyl, i-butyl, sec.-butyl, tert.-butyl. This also applies for more complex radicals like alkoxy or haloalkyl etc. If alkyl is not defined lower alkyl groups e.g. with 1—6 carbon atoms are preferred, especially alkyl groups with 1—4 carbon atoms. Alkyl $C_1$—$C_5$ also includes pentyl, i-pentyl etc. If halogen is not specifed it is F, Cl, Br or J.

The synthesis of the compounds, endowed with an insecticide and acaricide activity is achieved, for instance, by converting a cyclopropanecarboxylic ester or acid of formula I, in which *R* is respectively equal to O-alkyl $C_1$—$C_4$ or to OH, to the correspondent acyl-halide (I, R =halogen) and by reacting this latter with alcohols of the formula:

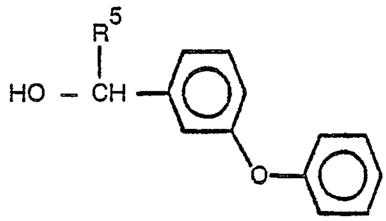

(II)

($R^5$ has the meanings reported in previous general formula I).

As far as the preparation of the new intermediate of general formula I is concerned, different procedures are possible, whose choice depends mainly on the nature of the substituents present in the dienic chain ($R^1$, $R^2$, $R^3$ and $R^4$).

In the following will be described: a process for the synthesis of the compounds of general formula I, wherein $R^4$ is different from H (method A); a process for the synthesis of the compounds of formula I wherein $R^3$ = H (method B), a process for the synthesis of the compounds of formula I wherein $R^4$ = H (method C), and an alternative process for the preparation of the compounds of formula I wherein $R^3$ = H (method D).

METHOD A:

A lower alkyl ester of 2,2-dimethyl-3-formylcyclopropanecarboxylic acid (1) (caronic aldehyde), as a mixture of geometric isomers or as a single isomer, is made to react according to the Wittig reaction with the ylide (2) of a haloalkenyl of formula (3):

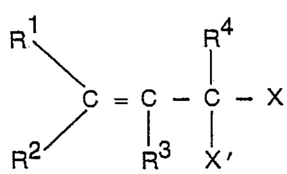

(3)

wherein: $R^1$, $R^2$ and $R^3$ have the previously specified meanings; $R^4$ = F, Cl, Br, $CF_3$; X and X′ (equal to or different from each other) = Cl, Br.

SCHEME 1:

1) (3)

2) (2)

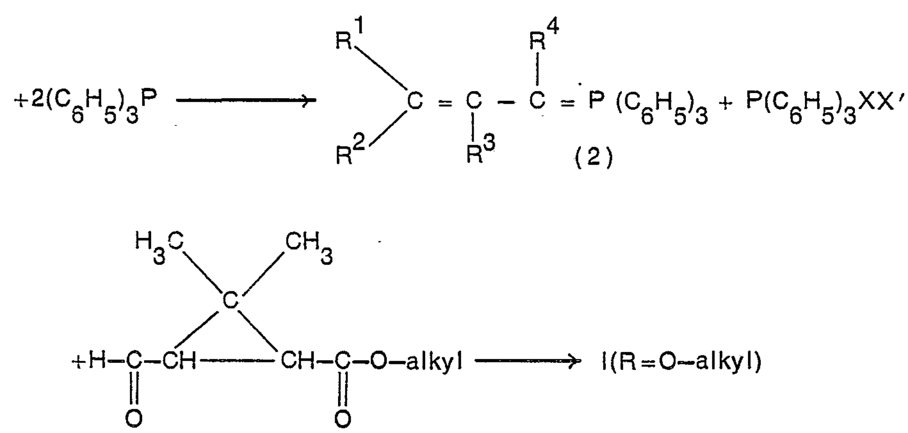

(1)

As examples of such compounds of formula (3), there may be cited:

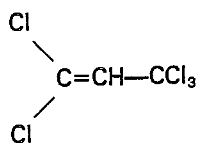

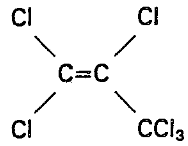

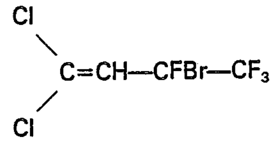

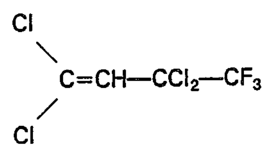

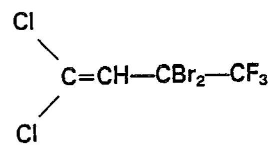

Some of the compounds of formula 3, and more particularly those of the general formula:

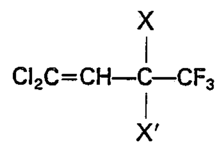

(III)

wherein: X and X′ (equal to or different from each other) = halogen, are new compounds.

Their preparation is achieved by dehydrohalogenation of 1,1,1-trifluoro-2,2,4,4,4-pentahalobutanes described in the following.

Alternatively to the process indicated in *scheme 1:* the compounds of formula I, wherein $R^4$ is a halogen atom, may be prepared by carrying out a Wittig reaction between a lower alkyl ester of 2,2-dimethyl-3-formyl-cyclopropanecarboxylic acid (1) and a polyhaloalkane of formula (4):

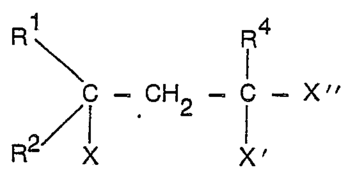

(4)

and by subjecting the resulting product of this reaction to a dehydrohalogenation with bases, according to Scheme 1b.

SCHEME 1b

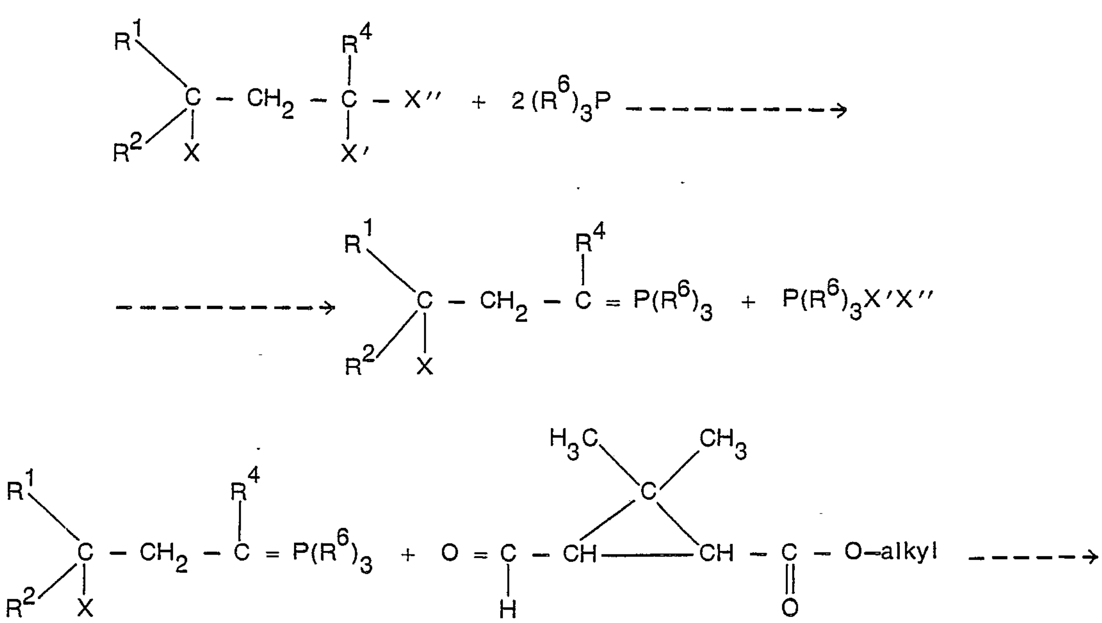

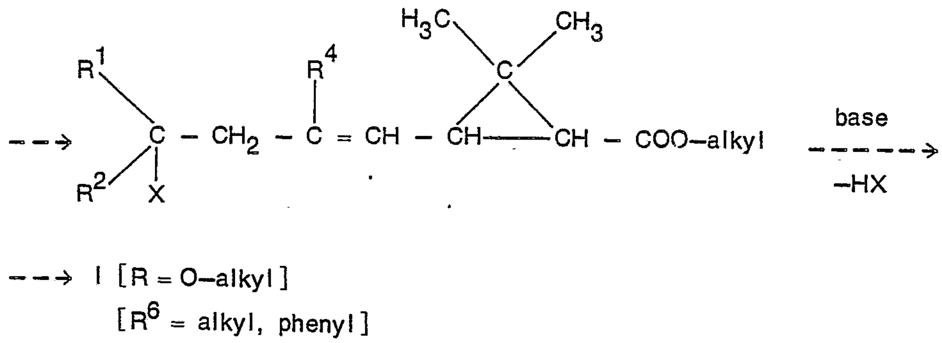


As a further alternative to the process indicated in *scheme 1,* the compounds of formlua 1, wherein $R^4$ is either a chlorine or bromine atom, may be prepared by a Wittig reaction between allyl chloride or allyl bromide of the type:

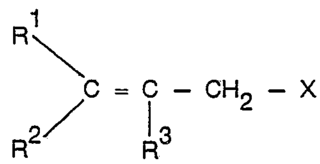

and a lower alkyl ester of 2,2-dimethyl-3-formyl-cyclopropanecarboxylic acid (1) and by then subjecting the therefrom resulting dienic product to a halogenation with $Cl_2$ or $Br_2$, followed by dehydrohalogenation.

SCHEME 1 c:

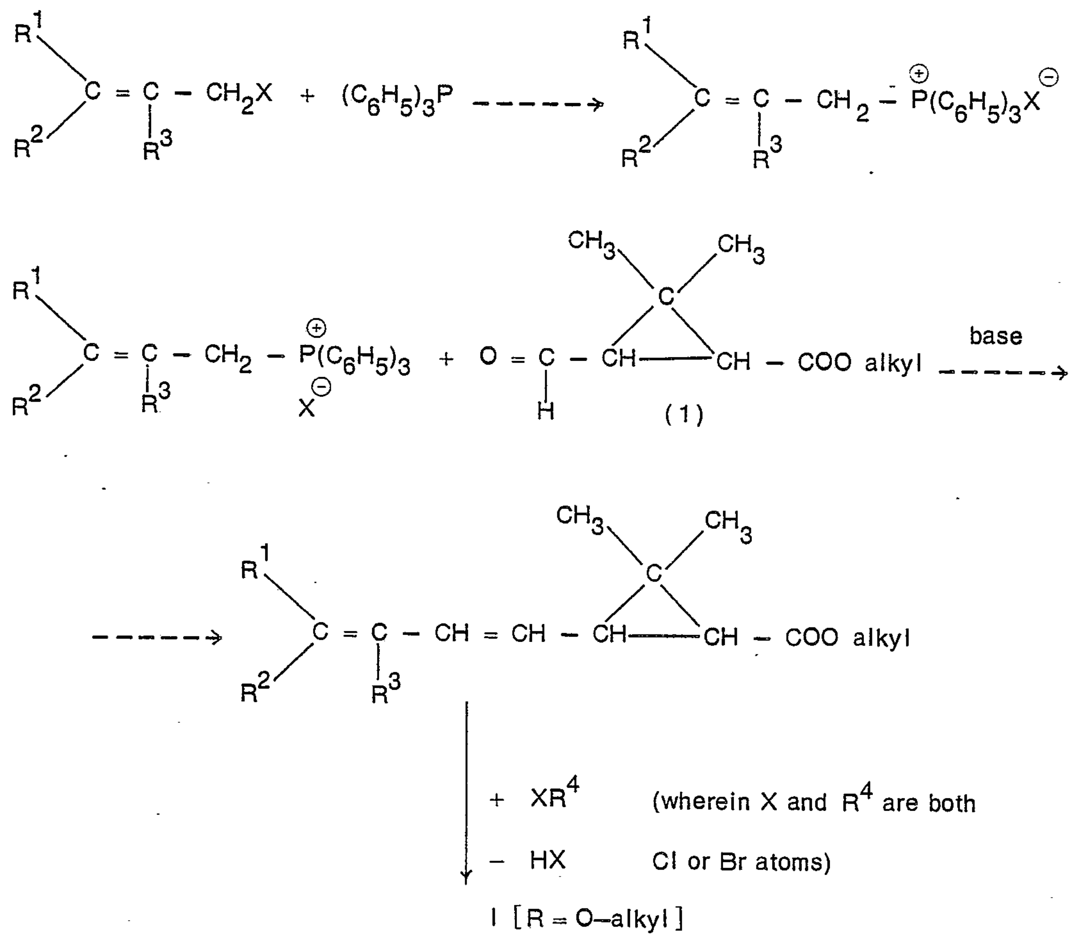

METHOD B

A polyhaloalkane (propane or butane) (4) is additioned with an ester of 3,3-dimethyl-4-pentenoic acid (5, n = 1) or 2-ethoxycarbonyl-3,3-dimethyl-4-pentenoic acid (5, n = 2):

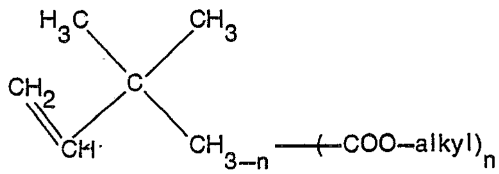

(5)

(n = 1 or 2)

thereby obtaining adduct (6) which, treated with bases, undergoes in one or more stages, successive dehydrohalogenations with the elimination of three mols of a halogenhydric acids optionally followed (in the case n = 2) by a decarboxylation.

In the case the dehydrohalogenation is carried on and $R^2$ is a halogen atom, there are obtained the compounds of formula I in which $R^2$ and $R^3$ together constitute a third bond.

SCHEME 2:

3) 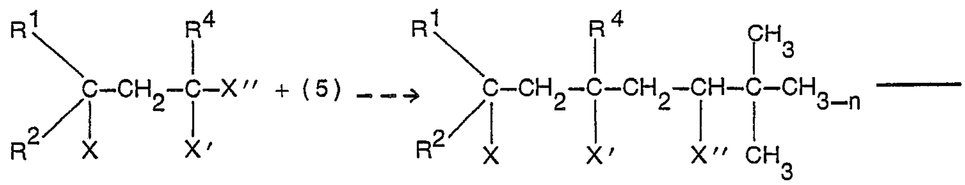

(4) (6)

—— $(COO{-}alkyl)_n$

4) (6) 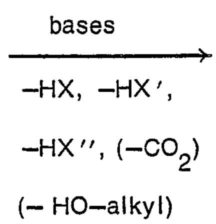

I (R = OH, O–alkyl; $R^3$ = H)

[X, X′ and X″ (equal to or different from each other) = Cl, Br; n = 1 or 2; $R^1$, $R^2$ and $R^4$ have the same meanings as those reported in general formula I].

The polyhaloalkanes of formula (4), suited for reacting according to reaction 3), are, for instance: 1,1,1,3,3,3-hexahalopropanes, 1,1,1,3,3,-pentahalobutanes, 1,1,1,2,2,4,4,4-octahalobutanes.

Amongst these compounds there may be cited a few as examples:

$CCl_3—CH_2—CCl_3$
$CH_3—CCl_2—CH_2—CCl_3$
$CF_3—CCl_2—CH_2—CCl_3$
$CF_3—CBr_2—CH_2—CCl_2Br$
$CF_3—CFBr—CH_2—CCl_2Br$.

The 1,1,1-trifluoro-2,2,4,4,4-pentahalobutanes (IV), are new compounds and were obtained by reacting a polyhaloethane of formula (7):

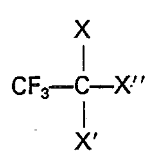

(7)

with vinylidene chloride, according to equation 5):

5)

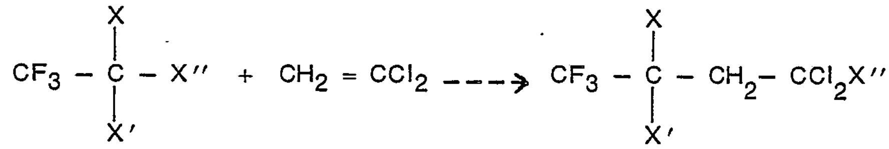

(IV)

[wherein X = F, Cl, Br; X' and X'' (either equal to or different from each other) = Cl, Br].

Both reactions 3 and 5, in general, are achieved by reacting the halo-alkane (4 or 7) with the unsaturated ester (5) or with the vinylidene chloride, in a molar ratio from 1 to 4.

The above indicated reactions are conducted in inert solvents at a temperature comprised between 50° and 200°C and in the presence of catalytic amounts of radical reaction promoters, such as organic peroxides (ter-butyl-peroxide, benzoyl-peroxide, diacetyl-peroxide), azo-derivatives (azo-bis-isobutyronitrile), U.V. radiation, or in the presence of redox-transfer systems or of metal-carbonyls, etc.

Reaction 4 is conducted according to known techniques, either in one single or in plurality of successive stages, using organic bases (amines), alkaline alcoholates or inorganic bases (NaOH, KOH).

METHOD C:

A mixture of cis and trans isomers of esters with lower alcohols of 2,2-dimethyl-3-acetoxymethyl-cyclopropanecarboxylic acid (8) is hydrolyzed with catalytic amounts of sodium ethylate in ethanol, thereby obtaining a mixture of ethyl ester of trans 2,2-dimethyl-3-hydroxymethyl-cyclopropane-carboxylic acid (9) and of the lactone of the same acid in the cis form (10).

Compounds 9 and 10 may be separated by distillation, and each one may be independently converted into the corresponding 3-bromoethyl derivative, i.e.: compound 9 by treatment with triphenylphosphine bromide $(C_6H_5)_3PBr_2$ and compound 10 by treatment with HBr, thereby obtaining respectively the 3-bromomethyl-derivative of trans ethyl ester (11-trans) and the 3-bromomethyl-derivative of the cis acid (12) which will then be converted to the corresponding alkyl ester (11-cis).

Intermediates 11-cis and -trans, either separately or in admixture with each other, are converted to the corresponding phosphonium salts (13) by treatment with triphenylphosphine $(C_6H_5)_3P$.

Finally the phosphonium salts (13) are made to react with an aldehyde of the formula:

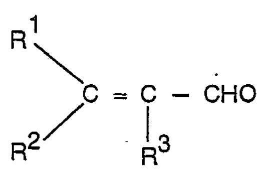

(14)

($R^1$, $R^2$ and $R^3$ have the same meanings indicated for the general formula I).

Thereby are obtained the compounds of formula I wherein: R = O-alkyl and $R^4$ = H.

SCHEME 3:

6)

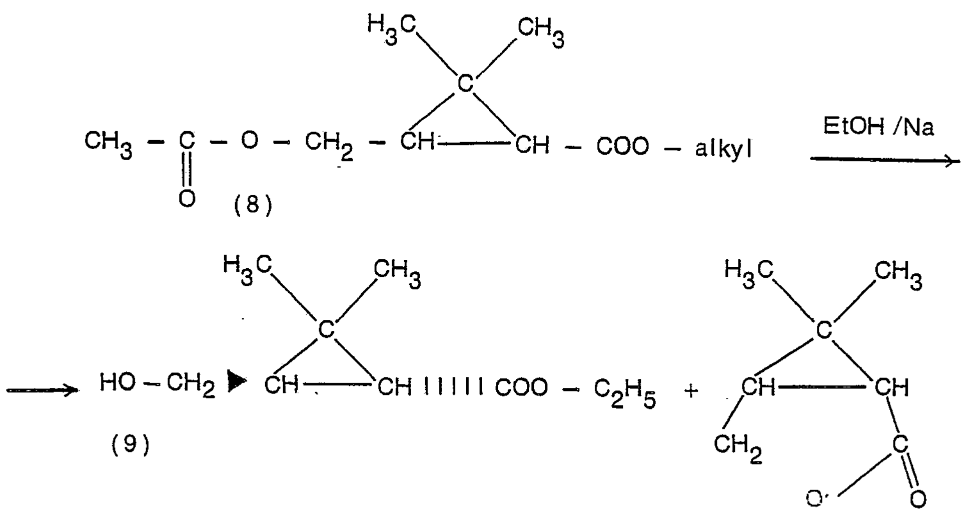


(8)

(9)

(10)

7) (9) 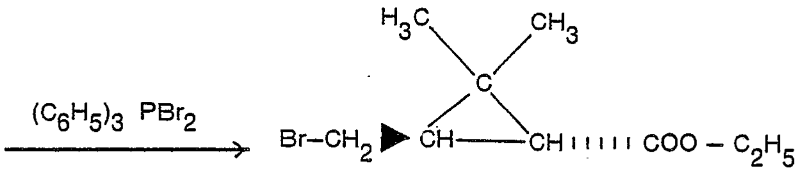


(11–trans)

8) (10) 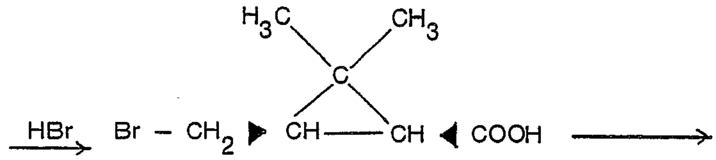


(12)

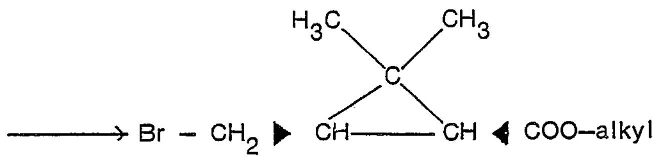

(11–cis)

9) (11) + 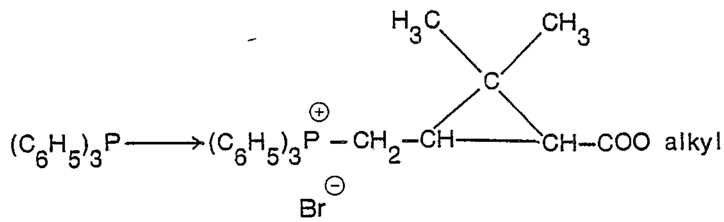

(13)

10) (13') + 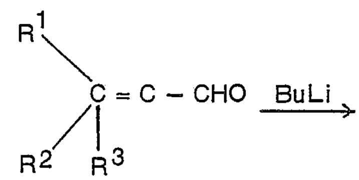

I (R = O–alkyl, $R^4$ = H)

(14)

The reactions from 6) to 9) are carried out by applying well known techniques in the transformations of hydroxy acid esters by hydrolysis and lactonization, in the substitution of an OH group by a bromine atom and in the formation of phosphonium salts from bromo-alkyl derivatives. The examples given in the present patent application give an adequate documentation of the sequence of these reactions.

Wittig reaction 10), although known by itself, has never been applied to haloganted derivatives of acrolein. This reaction is conducted by suspending the phosphonium salt (13) in an anhydrous inert solvent (e.g.: tetrahydrofurane), in an anhydrous and deoxigenated nitrogen atmosphere according to what described by L.F. Fieser in: "Reagents for Organic Synthesis" — J. Wiley & Sons Ed., 1967, page 149. To this suspension is then admixed a stoichiometric quantity of butyl-lithium dissolved in the same solvent, at a temperature comprised between −70° and −20°. As soon as the butyllithium has disappeared, [as can be checked by carrying out the Gilman test described in: "Organic Reactions", *6*, (1951) page 352] to the ylide thus prepared is added the aldehyde (14) in slight excess and is then allowed to react at a temperature of between −20° and 0°C. Once the reaction has ended, the betainic complex that has formed is preferably decomposed by treatment with a stoichiometric quantity of phosphoric hexamethyltriamide and then with water, in order to obtain the dienic I (R = O-alkyl, $R^4$ = H).

The esters with lower alcohols of 2,2-dimethyl-3-acetoximethyl-cyclopropanecarboxylic acid (compound 8) have been described in DE—OS 30 26 011.

As examples of aldehydes of formula (14), suited for the reaction with phosphonic salts (13) according to the Wittig reaction (reaction 10), there may be cited:

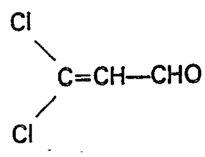
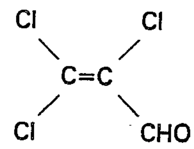
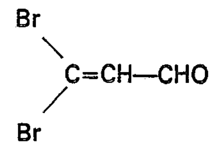
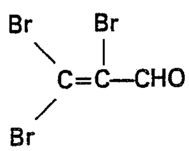
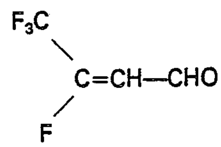
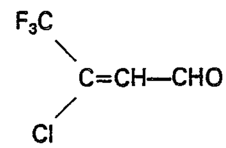
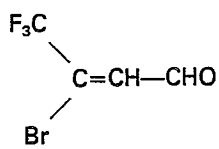
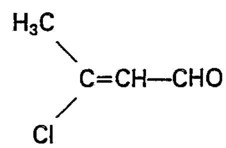

Some of the aldehydes of formula 14, and more particularly, those having in β a $CF_3$ group and one halogen, of formula:

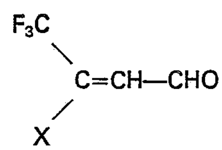
(V)

(X = F, Cl, Br) are new compounds.

Their preparation is achieved by reacting 1,1,1-trifluoro-2,2,2-trihaloethane with a vinylether followed by dehydrahalogenation and hydrolysis.

The addition reaction of 1,1,1-trifluoro-2,2,2-trihaloethane to a vinylalkylether (or to another vinyloxiderivative, such as for instance vinyl acetate) is conducted in the presence of radical reactions promoters, at temperatures of between 0° and 200°C, by operating with an excess of polyhaloethane, with or without solvents.

As a radical reaction promoter there may be used one of the following agents: U.V. light, peroxides, transition metal salts such as for instance iron or copper salts, in the presence of either primary or secondary amines, or azo-derivatives such as for instance azo-bis-isobutyronitrile.

11)
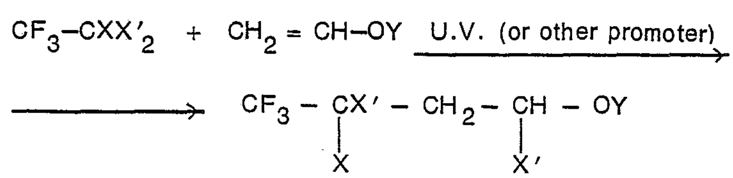


wherein: X = F, Cl, Br; X′ = Cl, Br; Y = an alkyl group with from 1 to 5 carbon atoms, or

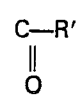

(R′ = lower alkyl).

Some further examples of the addition of other fluoro alkanes to vinyl ethers have been reported by P. Tarrant, E. C. Stump in the Journal of Organic Chemistry, *29*, p.1198 (1964).

Once there has been obtained the adduct, this is subjected to dehydrohalogenation and hydrolysis of the α-haloether group by treatment with boiling water or with bases or salts developing an alkaline action (e.g.: $Na_2CO_3$, $NaHCO_3$) in an aqueous medium (equation 12).

Alternatively, the dehydrohalogenation reaction may be carried out in a stage preceding the hydrolysis, for instance by heating up the adduct to a temperature at which there is a thermal elimination of the hydrohalic acid HX (equation 14).

In its turn, the conversion of the α-haloetheric group (when R = alkyl) to an aldehydic group may be achieved by pyrolysis (equation 15) instead of by hydrolysis (equation 13).

A further alternative consists in conducting the hydrolysis reaction in the presence of water, at the boiling temperature (equation 16), followed by a dehydrohalogenation in the presence of an aqueous base (equation 17).

12)

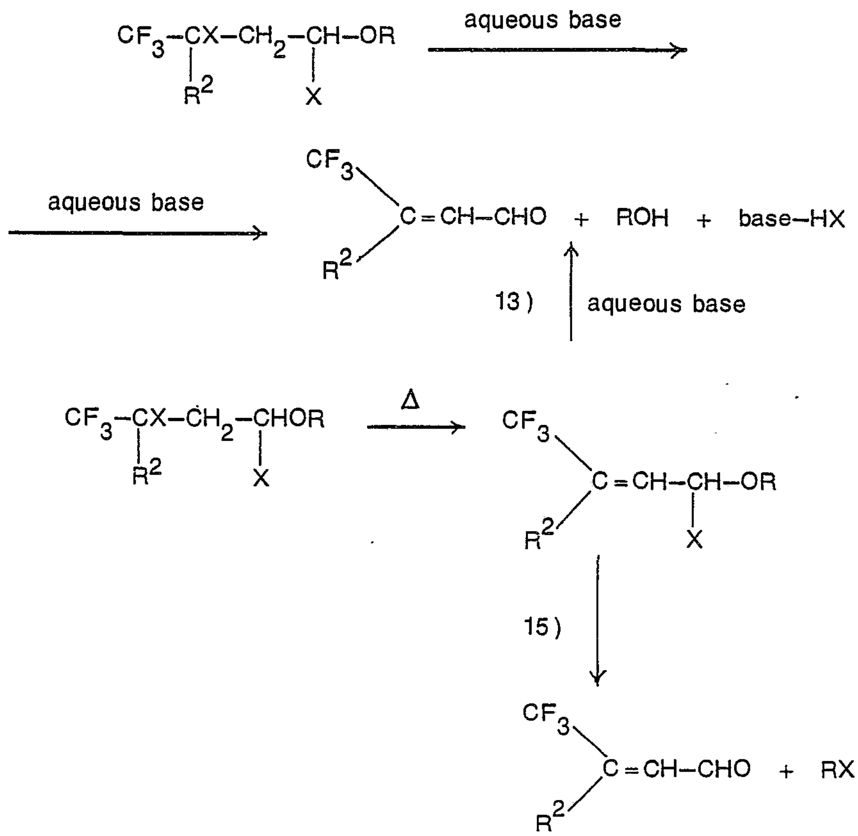


13)

14)

15)

16)

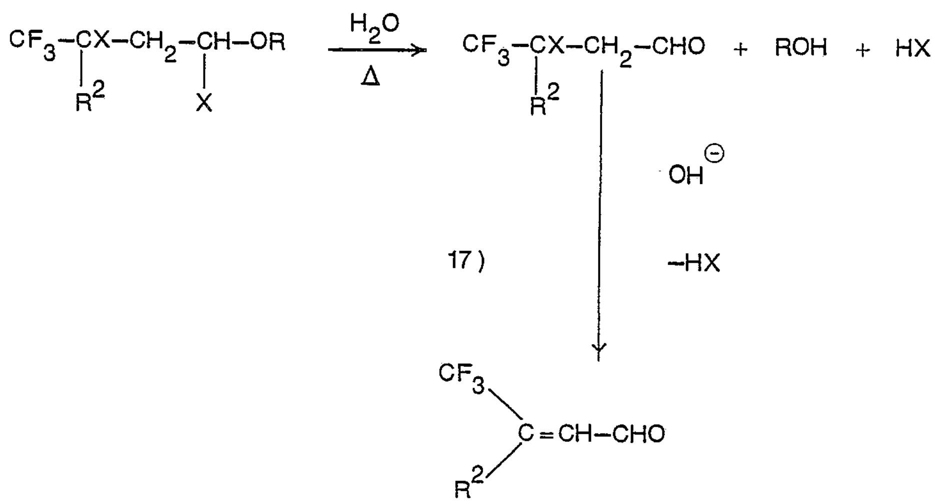


17)

The thermal decomposition reactions (in the absence of water) are conducted at temperatures comprised between 70°C and 250°C.

Method D:

Consists in a synthetic procedure alternative to *Method B* for the preparation of the compounds of formula I in which R = O-alkyl, $R^3$ =H and $R^4$ = H, F, Cl, Br.

The process reported under Method D results to be more convenient than that of Method B in as much as it is of easier execution affording higher yields. Moreover the products are isolated from the reaction mixture in a simple way and with a high degree of purity.

Because of the fact that the substituent $R^4$ values in the compounds which can be prepared by Method D is slightly limited with respect to the values of $R^4$ in the compounds which can be prepared by Method B,

and in order to avoid misunderstandings, in the description of Method D, the substituent $R^4$ will be replaced by $R^6$.

Thus, the process of Method D is useful for the preparation of some of the compounds of general formula I, having the following general formula:

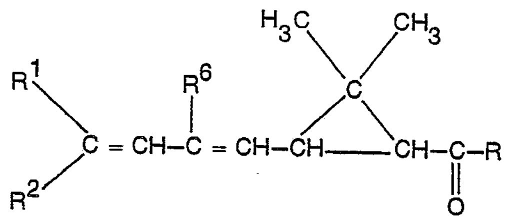

(VI)

wherein $R^1$ and $R^2$ have the meanings reported for general formula I, R = O-alkyl $C_1$—$C_4$ and $R^6$ = H, F, Cl, Br. Said process consists in reacting, in the presence of a strong base, a 2-alken-phosphonate of the formula:

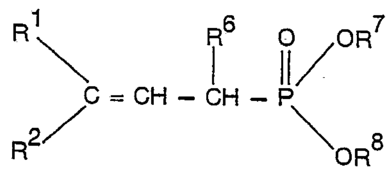

(VII)

[wherein $R^1$, $R^2$ and $R^6$ have the meanings given to them in formula VI; $R^7$ and $R^8$ (equal to or different from each other) represent an alkyl or a haloalkyl with $C_1$—$C_4$, or $R^7$ and $R^8$ together form either a

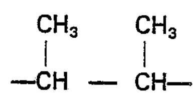

or a

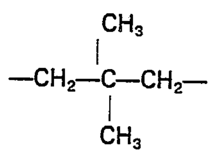

group] with a lower alkylester or caronic aldehyde (1) (2,2-dimethyl-3-formylcyclopropanecarboxylic acid) of the formula:

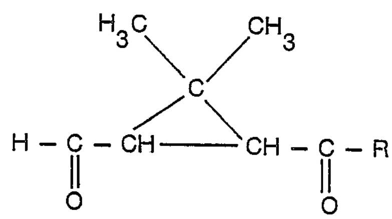

(1)

(wherein: R = O-alkyl $C_1$—$C_4$).

The above process, is illustrated by reaction 18):

18)

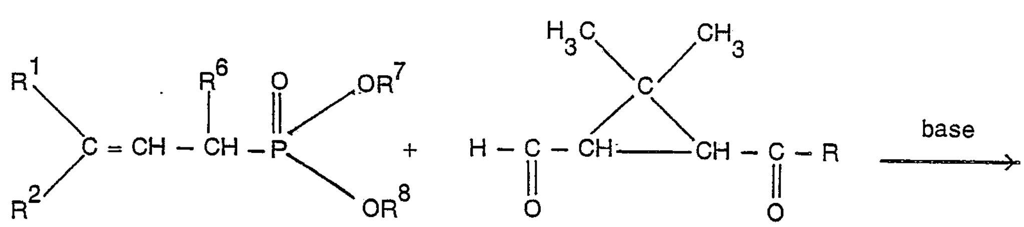


(VI)

(1)

The compounds of formula VII have been indicated as phosphonates, thereby using this term in its more general meaning, that is, referred to organic compounds of the pentavalent phosphorus in which there is present a carbon-phosphorus bond.

It must, however, be pointed out that the correct nomenclature for the compounds of formula VII (in which substituents $R^7$ and $R^8$ together represent either a

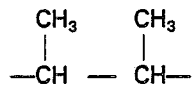

or a

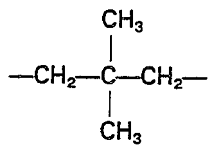

group and thus form with the

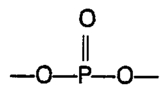

group either a pentaatomic or hexaatomic ring) foresees for these compounds the term 2-oxo-1,3,2-dioxaphospholane and respectively 2-oxo-1,3,2-dioxa-phosphorinane.

In this context, the compounds of formula VII will in the following be indicated by the term "phosphonates", also comprising thereby the compounds of formula VII in which there occurs either a penta- or hexatomic ring.

As far as we know, only one example of a reaction between a 2-alken-phosphonate and an aldehyde was known from the literature.

Said example concerns the reaction between methyl 3-chloro-2-butene-phosphonate and benzaldehyde [G. Lavielle, C.R. Acad. Sci., Ser. C, 86 (1970)].

The above cited reaction leads to the formation of three products, only one of them having a dienic structure:

19)

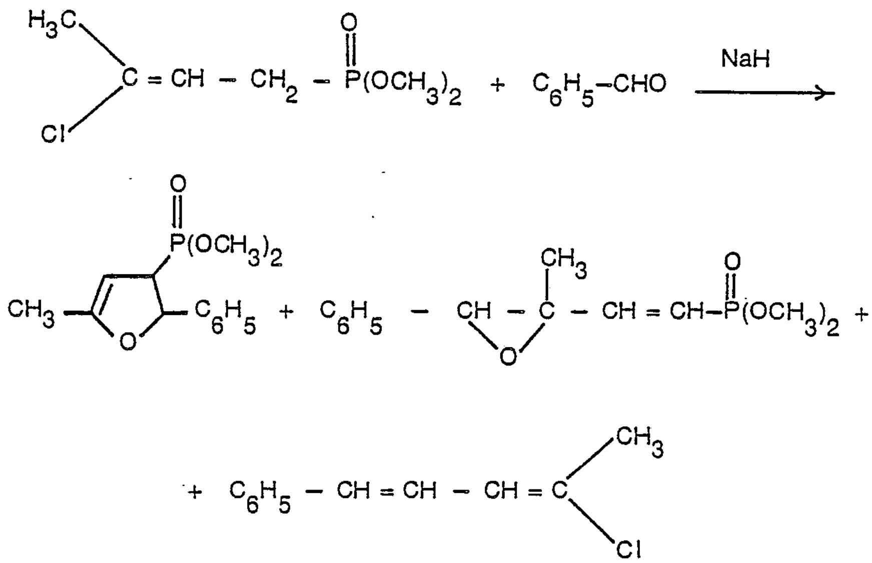


It had thus to be expected that also from reaction 18 there would form compounds of little interest for the purposes of the desired structure.

On the contrary, we surprisingly found that reaction 18 affords compounds of formula VI with high yields and allows to separate (isolate) the desired product quite easily and with a high degree of purity.

Reaction 18 is carried out using substantially equimolar quantities of phosphonate VII and of the ester of caronic aldehyde (1), in the presence of substantially equimolecular amounts of a strong base, in an inert solvent or diluent and at temperatures comprised between −50°C and +100°C, but preferably comprised between −30°C and room temperature. Suitable strong bases may be hydrides or hydroxides of alkaline metals such as sodium hydride, lithium hydride, sodium hydroxide or potassium hydroxide.

In case that as a base there is used sodium hydride, it is preferable to operate in the presence of catalytic amounts of suitable activators such as for instance ethyl alcohol or imidazole. As a reaction medium there may be used an ether such as for instance: dimethoxy-ethane or tetrahydrofurane, an aprotic polar solvent such as N,N-dimethylformamide or a pair of protic-aprotic solvents such as e.g. water and an aromatic hydrocarbon (in this latter case, as a base there must be used an alkaline hydroxide).

In a practical embodiment, the reaction 18 may be conducted by preparing a mixture of phosphonate VII and of the ester of caronic aldehyde (1), in a molar ratio of about 1:1 and by then admixing slowly this mixture to a solution or a suspension of the base in a suitable inert solvent.

In another practical form of embodiment, reaction 18 is conducted by slowly admixing a solution of phosphonate VII in an inert solvent to a mixture consisting of the base, of the ester of caronic aldehyde (1) and of an inert solvent. Reaction 18, in general, is carried out in an anhydrous medium and under an atmosphere of an inert gas when a base there is used an alkaline metal-hydride. The lower alkyl esters of caronic aldehyde (1) are known compounds that are separable in two isomeric forms, cis and trans (isomerism on the cyclopropane ring).

The possibility to use in reaction 18 also only just one of the two cis or trans forms, allows to obtain cyclopropanecarboxylic ester (VI) having the corresponding isomerism.

As far as is known to us, the only 2-alkene-phosphonate of formula VII known previously, is methyl 3-chloro-2-butene-phosphonate (reaction 19).

The preparation of the phosphonates of formula VII is carried out according to known techniques starting from a 2-alkenyl halide (15) and from a suitable phosphorus compound.

Reactions 20 to 27 described below are representative examples of methods for the preparation of the various phosphonates of formula VII.

20) 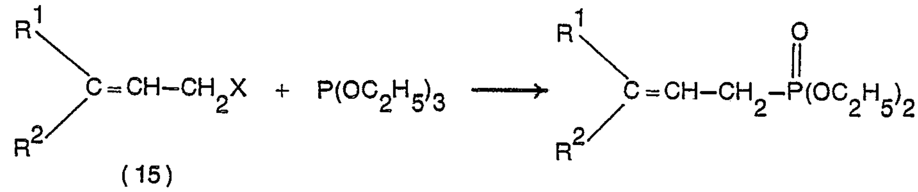

(15)

[VII, $R^6 = H$, $R^7 = R^8 = C_2H_5$]

21) (15) + 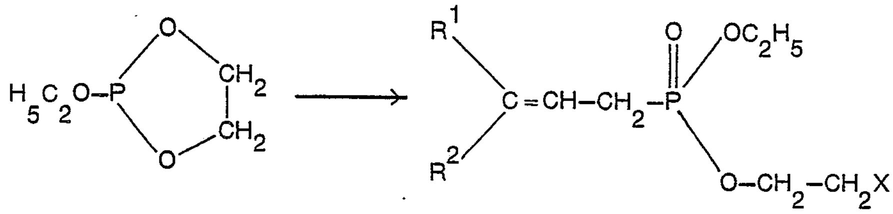

[VII, $R^7$ (or $R^8$) = haloalkyl]

22) (15) + 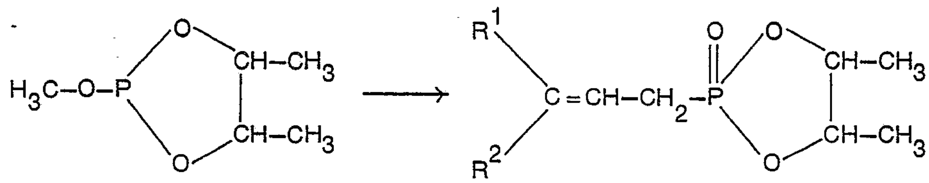

[VII, $R^7$ and $R^8$ together = $-\overset{CH_3}{CH}-\overset{CH_3}{CH}-$]

23) (15) + 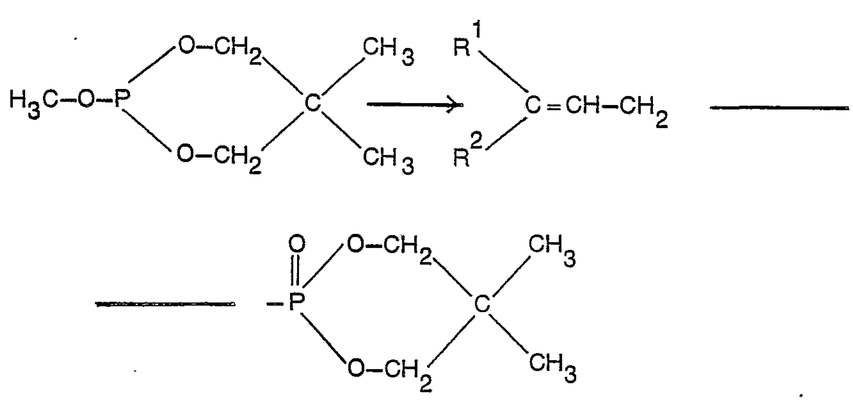

[VII, $R^7$ and $R^8$ together = 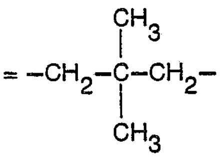 ]

24) [VII, $R^6$ = H] 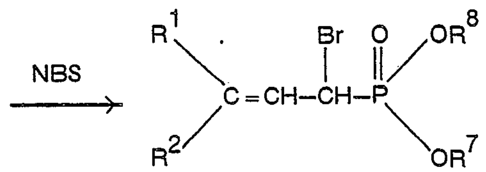


[VII, $R^6$ = Br]

25) [VII, $R^6$ = H] 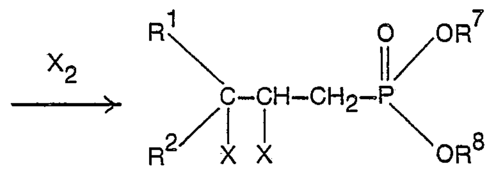

(16)

26) (16) 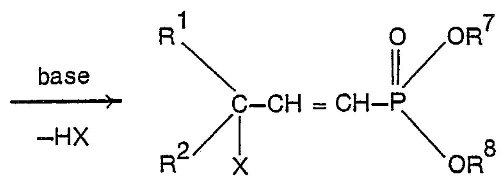

(17)

27) (17) 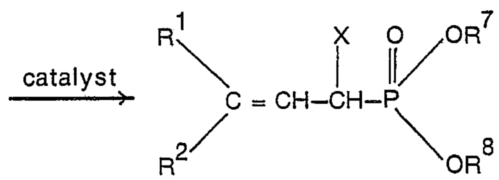

[VII, $R^6$ = halogen]

In the above reported reaction $R^1$, $R^2$, $R^6$, $R^7$ and $R^8$, if not otherwise indicated, have the meanings reported for formula VII; *X* stands for chlorine or bromine while NBS indicates the N-bromo-succinimide compound. Reactions from 20 to 23 represent examples of methods for the preparation of the compounds of formula VII wherein $R^6$ = H. The compounds wherein $R^6$ = halogen, may be prepared in a similar way starting from suitable 2-alkenyl halides, or they may be prepared from the compounds of formula VII wherein $R^6$ = H, by the methods illustrated by reactions 24 to 27.

All starting compounds of reaction 20 to 23 are known compounds or they can be easily prepared according to known methods. Reaction 24 concerns a standard halogenation in allylic position, carried out with N-bromo-succinimide.

Reactions 25 to 27 represent an alternative to reaction 24, also useful for preparing compounds in which $R^6$ = Cl. The addition of halogen ($X_2$) to compounds VII wherein $R^6$ = H(reaction 25) is carried out according to techniques that are standard in organic chemistry, using halogens or halogenating agents such as $SO_2Cl_2$.

The dehydrohalogenation of adduct (16) (reaction 26) may be carried out either in the presence of inorganic bases or of organic bases such as secondary or tertiary amines.

The reaction of the re-arrangement of adducts (17) (reaction 27) is new and not yet described.

Said reaction occurs quantitatively at a temperature comprised between 20° and 150°C, in the presence of catalytic quantities of a bivalent copper halide, by simple stirring of the mixture consisting of the adduct (17) and of cupric halide.

In case X = Cl, a suitable catalyst is cupric chloride in the commonly used form of dihydrate.

By means of the hereabove described methods A to D, the compounds of formula I in which R = OH or O-alkyl can be prepared.

From these compounds, with methods known in the common practice of organic chemistry, it is possible to prepare the acylic halides (I, R = halogen), for instance, by the reaction of the acids with thionyl chloride.

The compounds of formula I, wherein:

$R =$ 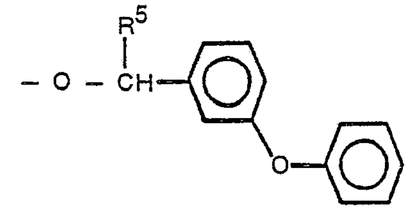

were obtained by reacting these acylic halides with alcohols of General Formula II, according to known techniques.

The compounds of general formula I, in general are obtained as mixtures of geometrical and configurational isomers due to the particular structure of the molecule which contains asymmetric carbon atoms and double bonds.

The separation of the various mixtures in the various diastereoisomers may be achieved following the techniques usually applied in the normal practice of organic chemistry, such as for instance chromatographic methods.

The separation and the use of the single isomers of their mixtures obtained by partial separation of the isomers of the compounds of general formula I are comprised by the present invention.

The compounds of general formula I, wherein R is an alcoholic residue of the 3-phenoxy-benzyl alcohol optionally substituted, display a high insecticide activity against insects belonging to the most important species, from the point of view of their noxiousness in the agricultural and civil fields, such as hemiptera, lepidoptera, coleoptera and blattoidea, and possess, moreover, a high acaricide activity, particularly as ovicides.

In this latter action they prove by far more active than the best pyrethroids known.

Moreover, they are endowed with a high stability to photo-oxidation, a property that ensures a sufficient persistence of their action thus allowing also their use in agriculture.

The insecticide compounds of general formula I may be applied to a zone where insect control is desired both as technical materials or as suitable compositions of formulations.

Suitable compositions comprise an insecticide compound of formula I as active ingredient in combination with one or more suitable inert carriers and/or surface active agents, and optionally other active compounds such as other insecticides, acaricides or nematocides.

Suitable formulations include granules, dusts, wettable powders, emulsifiable concentrates, solutions, dispersions and the like.

The active ingredient may be present in a suitable composition at a concentration of from 0.1% to 99% by weight.

While the application rate of the formulations varies widely depending on the type of formulation, the active compound, the mode of application and the environment, an effective insecticidal amount of the active principle must be applied and the practical rate may vary in the range of 0.01 to 3 Kg/ha.

In order to even further illustrate this invention, in the following are given a number of examples.

## Example 1

a) Preparation of: $CF_3—CBr_2—CH_2—CCl_2Br$.

Into a glass flask of 500 ml holding capacity, fitted with a reflux condenser, there were introduced, under a nitrogen atmosphere, the following reactants:

339 g of 1,1,1-trifluoro-2,2,2-tribromethane (1.056 mols),
51 g of 1,1-dichloroethylene (0.526 mols),
0.5 g of CuCl and
10 ml of ethanolamine.

This reaction mixture was then heated up at the reflux temperature for 4 hours.

After cooling down, the reaction mixture was washed with water until attaining a neutral pH, whereupon it was subjected to distillation.

After removal of the excess trifluorotribromoethane as a head, there were gathered 30 g of a fraction with b.p. comprised between 43° and 45°C at 0.2 mbar, consisting for 90% of it of 1,1,1-trifluoro-2,2,4-tribromo-4,4-dichlorobutane, as evidenced by mass spectrometry combined with gas-chromatographic separation.

Mass spectrum:
peak of molecular ion:

$$C_4H_2F_3Br_3Cl_2$$

main fragments:

$$CF_3—CBr_2^+$$
$$CCl_2Br^+$$

and for 10% of it of the dehydrohalogenation product:

Mass spectrography:

peak of molecular ion:

$$C_4HF_3Br_2Cl_2.$$

b) Preparation of ethyl 3,3-dimethyl-4,8,8-tribromo-6,6-dichloro-9,9,9-trifluorononanoate.

$$CF_3—CBr_2—CH_2—CCl_2—CH_2—CHBr—C(CH_3)_2—CH_2—COOC_2H_5.$$

Into a quartz vial were introduced, under an atmosphere of nitrogen:

— 41.8 g of $CF_3—CBr_2—CH_2—CCl_2Br$ (prepared as described in part a),

— 15.6 g of ethyl 3,3-dimethyl-pent-4-enoate.

The vial was then sealed and irradiated with a high pressure Hanau lamp for 15 hours at 65°C. The content of the vial was then distilled in order to remove the unconverted reactants.

The residue (12 g) proved to consist essentially of the desired product (gas-chromatographic analysis).

Mass fragmentations:

| | |
|---|---|
| $C_{13}H_{18}F_3Cl_2Br_3O_2$ | $M^+/e$ = 269, 189, 161, 155 (main peak) 135, 129, 109, 88, 87. |

c) Preparation of ethyl 3-(2′-chloro-4′-bromo-4′-trifluoromethyl-butadienyl)-2,2-dimethyl-cyclopropanecarboxylate.

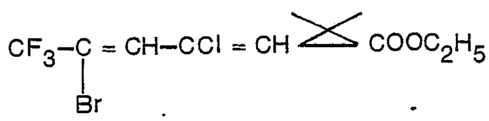

The adduct prepared as described in part b) (0.02 mols), was treated with 0.06 equivalents of sodium ethylate in 40 ml of absolute ethyl alcohol at a temperature of 60°C for 2 hours.

After removal of the solvent, the residue was diluted with methylene chloride, then washed with water, acidified with acetic acid, until obtaining a neutral pH, then anhydrified on $CaCl_2$ and then, after removal of the solvent, subjected to fractioned distillation gathering the fraction that boils at 62°—63°C at 0.27 mbar.

This fraction proved to consist for about 65% of the desired product.

Example 2: (method B)

a) Preparation of ethyl 3,3-dimethyl-4,6,6,8,8,8-hexachloro-octanoate.

$$CCl_3—CH_2—CCl_2—CH_2—CHCl—C(CH_3)_2—CH_2—COOC_2H_5.(1)$$

Into a 50 ml flask, fitted with a stirrer and a reflux condenser, connected at the upper end to a calcium chloride valve, there were introduced:

12.5 g of 1,1,1-3,3,3-hexachloropropane (0.05 mols),

8 g of ethyl 3,3-dimethyl-pent-4-enoate (0.05 mols),

1 g of ditert.-butylperoxide.

The reaction mixture was then heated up to 150°C for 24 hours. After cooling down, from the mixture was drawn a sample of the product which, by gas-chromatographic analysis, proved to consist for about 50% of it of the addition product and of minor quantities of hexachloropropane and of ethyl 3,3-dimethyl-pent-4-enoate that had not reacted. These latter were thereupon removed from the reaction mixture by distillation under vacuum, thereby gathering the fractions that boil until a temperature of the vapors of 110°C at 0.67 mbar.

The residue (11 g) was extracted with n-hexane, then purified with active charcoal and subjected to the removal of the solvent in order to give 7 grams of ethyl 3,3-dimethyl-4,6-6,8,8,8-hexachlorooctanoate.

Mass spectrometry:

($C_{12}H_{18}Cl_6O_2$)
404 ($M^+$)
359 ($M^+—OC_2H_5$)
333 ($M^+—OC_2H_5$, —Cl, —HCl)
287 ($M^+—CCl_3$)
129 ($C(CH_3)_2—CH_2—COOC_2H_5^+$)
117 ($CCl_3^+$)
87 ($CH_2—COOC_2H_5^+$)

b) Dehydrohalogenation of ethyl 3,3-dimethyl-4,6,6,8,8,8-hexachlorooctanoate (1).

To a solution of 2.1 g of (1), (prepared as described above) in anhydrous n-hexane (25 ml) there was added a solution of 2 grams of tetramethyl-guanidine in 25 ml of anhydrous n.hexane.

The reaction mixture was then maintained under stirring for 24 hours at room temperature, whereafter it was poured into cold water. The hexanic phase was thereupon separated and the aqueous phase was extracted with diethyl ether (50 ml). The etheric phase was recombined with the hexanic phase.

The resulting solution was washed with a diluted solution of HCl, until attaining a neutral pH. It was then anhydrified with $CaCl_2$ and then concentrated until reaching a constant weight thus obtaining a raw product (7 g) consisting of compound A (95%) and of compound B (5%).

Compound A: ethyl 3,3-dimethyl-4,6,8,8-tetrachloroocta-5,7-dienoate.

$$Cl_2C{=}CH{-}CCl{=}CH{-}CHCl{-}C(CH_3)_2{-}CH_2{-}COOC_2H_5$$

(mixture of cis and trans isomers).

The IR analysis is consistent with the assigned structure.

$^1$H NMR (δ, ppm):

| | |
|---|---|
| 1,2 | (9H, methyl groups) |
| 2,15—2,7 | (m, 2H) |
| 4,15 | (q,2H, O—$\underline{CH_2}$—$CH_3$) |
| 4,6—5,2 | (m, 1H) |
| 5,9—6,5 | (m, 2H) |

Mass fragmentation: ($M^+$/e)

| | |
|---|---|
| $C_{12}H_{16}O_2Cl_4$ | 332 ($M^+$)<br>296 ($M^+$—HCl)<br>260 ($M^+$—2HCl)<br>173 ($M^+$—3HCl, —$C_2H_5$)<br>96 |

Compound B: ethyl 2,2-dimethyl-3-(2′,4′,4′-trichlorobuta-1′,3′-dienyl)-cyclopropancarboxylate.

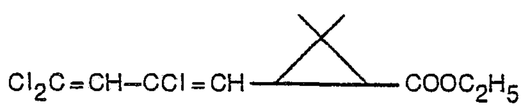

Mass fragmentation:

| | |
|---|---|
| $C_{12}H_{15}O_2Cl_3$ | 296 ($M^+$), 260 ($M^+$—HCl),<br>213 ($M^+$—$CHCl_2$)<br>177 ($M^+$—HCl,—$COOC_2H_5$) |

[an alternative synthesis of compound B is reported in Example 12].

Example 3: (method C)

a) Preparation of [(2-carbethoxy-3,3-dimethyl)-cyclopropyl]-methyl-triphenylphosphonium bromide. (2)

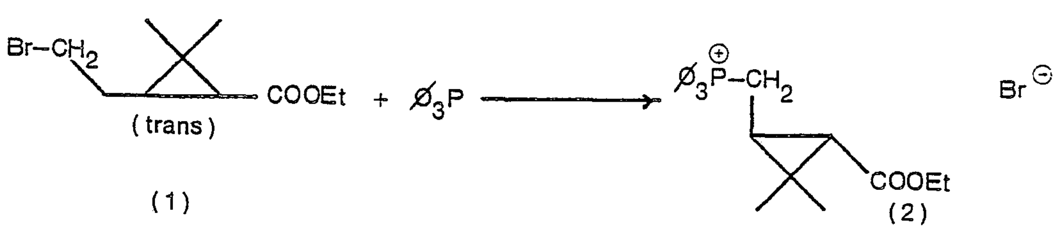

(trans)

(1)

(2)

Into a 2 litre autoclave were placed 0.163 mols of 2-bromomethyl-3,3-dimethyl-carbethoxy-cyclopropane-trans (1) (preparation see below) 0.18 mols of triphenylphosphine and 500 ml of ethyl ether. The autoclave was then washed with nitrogen, heated up to about 120°C (inside pressure about 9.3 bar) for 50 hours. The mixture was then cooled down and the white solid that was obtained was filtered. There were obtained 0.147 mols corresponding to a yield of 90%. The m.p. was about 182°—184°C.

The elemental analysis (in %ge):

C, theoretical: 64.56 C, found: 65.20
H, theoretical 6.05 H, found 6.08
Br, theoretical 16.07 Br, found: 16.25

b) Preparation of 3-(4′,4′-dichlorobutadienyl)-2,2-dimethylcarbethoxy-cyclopropane (trans) (3).

To a suspension of 0.01 mols of [(2-carbethoxy-3,3-dimethyl)-cyclopropyl]-methyl-triphenylphosphonium bromide (2) in 30 ml of tetrahydrofurane (distilled on lithium-aluminum-hydride), at −25°C, by dripping were admixed 0.01 mols of a butyl-lithium solution in hexane (1.5 molar).

The suspension was maintained under stirring for 20 min. at −20°C whereafter into it were dripped 0.014 mols of β,β-dichloro-acrolein (preparation see below) in 2 ml of tetrahydrofurane. Then, the temperature was allowed to rise to 0°C.

The mixture was maintained under stirring for 10 minutes and was then cooled down again to −20°C adding to it 0.01 mols of phosphoric hexamethyltriamide.

The mixture was then maintained under stirring at room temperature 20 minutes. To it was then admixed 10 ml of water and it was then kept under stirring for 2 hours. After this period, the mixture was poured into 50 ml of water, the phases were separated and the organic one was extracted with toluene (2 times, each time with 30 ml of solvent).

The organic phase was thereupon washed with water until attaining a neutral pH. Thereupon it was anhydrified on anhydrous sodium sulphate and evaporated.

Thereby were obtained 4.9 g of product (a dark oil) which was then passed through a cylindrical funnel of 5 cm diameter, containing 70 g of alumina. The mixture was then eluted with 100 ml of hexane (1st fraction), then with 200 ml of a mixture hexane-ethyl acetate (9:1) (2nd fraction) and finally with 300 ml of a mixture of hexane-ethyl acetate (7:3) (3rd fraction).

The three fractions were then evaporated separately. The 2nd (about 1.0 g) showed an I.R. spectrum coherent with the product. According to GLC there were present 2 isomers for a total of 50—55% of the fraction.

This product was then bubble distilled (temperature: 120°—140°C) at a pressure of 1.3 mbar, thereby obtaining the pure product.

I.R.: 1725, 1625, 1580 $cm^{-1}$;

NMR (δ, ppm, $CDCl_3$):

1.10—1.40 ($CH_3$)
1.50—1.70 (m, —CH—CO)
2.00—2.25 (m, —CH—C=C)
4.15 (q, $CH_2O$)
5.15—5.85 (m, <u>CH</u>=CH—CH=$CCl_2$)
6.0—6.5 (m, CH=<u>CH</u>—CH=$CCl_2$)
6.6—7 (m, CH=$CCl_2$).

[alternative synthesis of this compound are reported in Examples 14 and 15]

c) Preparation of the chloride of 3-(4′,4′-dichlorobutadienyl)-2,2-dimethyl-cyclopropanecarboxylic acid (trans), (5):

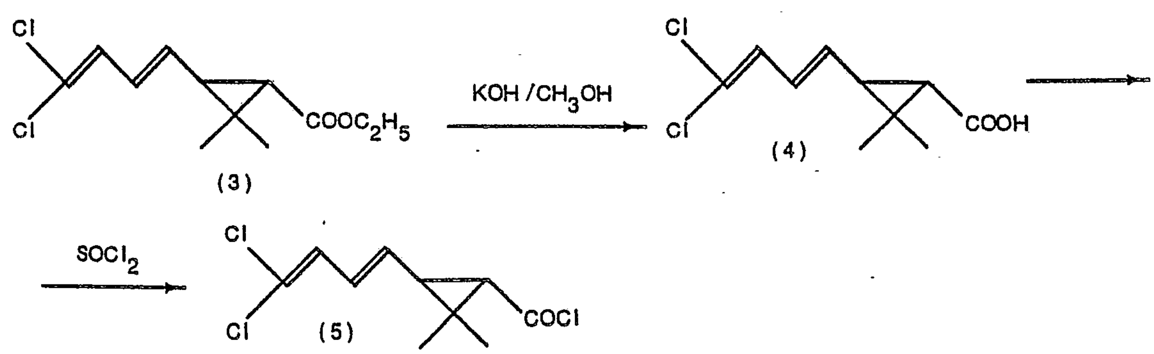


(3) (4) (5)

0.002 mols of the product (3), obtained as described part b), were treated with 2.6 ml of a 10% solution of KOH in methanol. It was then reflux heated for 4 hours by a water bath. Thereafter it was cooled down and then poured into 50 ml of water. It was then extracted with toluene (2 times, each time with 20 ml of solvent).

The organic phase was then washed with 20 ml of a 10% NaOH. The aqueous phases were then acidified with HCl at 5% concentration and then extracted with toluene (3 times, each time with 25 ml of solvent). The organic phase was thereupon washed with water until attaining a neutral pH, whereafter it was anhydrified on sodium sulphate and then evaporated, thereby obtaining 0.4 g of an oil consisting of the desired product (4).

I.R. ($cm^{-1}$): 3500—2500; 1700—1680; 1430; 1240; 1105; 890; 730; 690.

d) The raw 3-(4',4'-dichloro-butadienyl)-2,2-dimethyl-cyclopropanecarboxylic acid (4), obtained by means of the above described reaction, was dissolved in 3.5 ml of hexane. Then, at 0°—5°C, there were dripped in 3.5 m mols of thionyl chloride. This mixture was then subjected to reflux and stirring for 4 hours. Thereafter the hexane was decanted from the tars and evaporated at 40°C under a pressure of 20 mbar, thereby obtaining about 0.2 g of chloride of 2,2-dimethyl-3-(4',4'-dichlorobutadienyl)-cyclopropanecarboxylic acid (5).

| | | | |
|---|---|---|---|
| I.R., | v(C=O) | : | 1765 $cm^{-1}$ |
| | v($Cl_2C$=CH) | : | 1580 $cm^{-1}$ |
| | v(CH=CH) | : | 1615 $cm^{-1}$ |

e) Preparation of the α-cyano-3-phenoxybenzyl ester of the (±)-trans-3-(4',4'-dichlorobutadienyl)-2,2-dimethyl-cyclopropanecarboxylic acid [compound No. I]

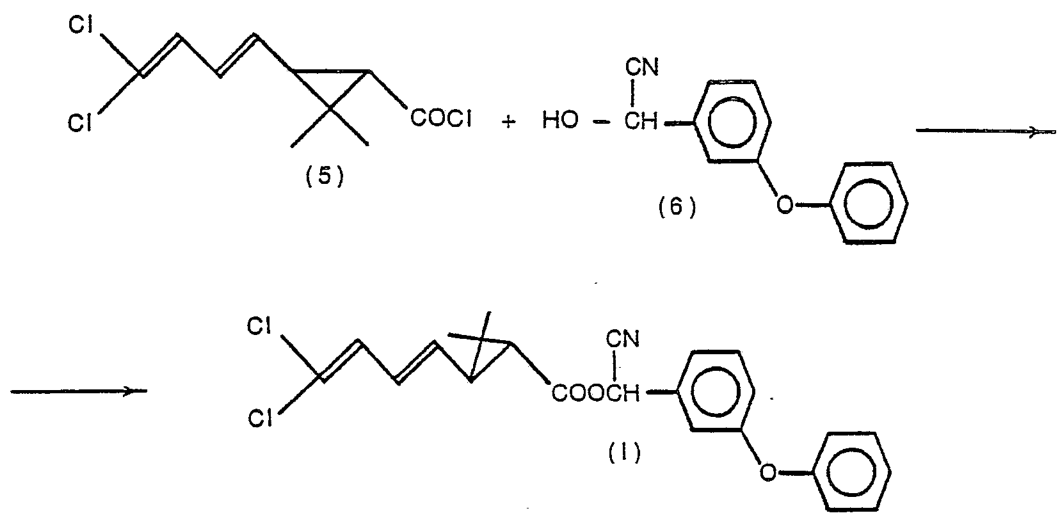

(5)

(6)

(I)

0.2 g of the acylic chloride (5), prepared as described in parts c) + d), were diluted with 1 ml of anhydrous ethylether. This mixture was cooled down to 0°C and then there was dripped into it a mixture of the cyanhydrine of phenoxybenzaldehyde (6) (180 mg = about 0.8 m mols) in 0.5 ml of anhydrous ether. This mixture was subjected to stirring at room temperature for 1 hour, whereafter it was cooled down to 0°C and into it was dripped a mixture consisting of 0.75 mols of pyridine and 0.5 ml of ether. Thereupon the temperature was allowed to slowly rise. The mixture was then maintained under stirring at room temperature overnight.

The mixture was then diluted with 5 ml of benzene, washed with water up to a neutral pH (2 × 5 ml), anhydrified, filtered and evaporated whereby there was obtained an oily residue of about 400 mg. Thereupon was rapidly carried out a chromatography with 20 g of silica, eluting with hexane-ethyl acetate (9:1). There were obtained 200 mg of a pure product (I).

GLC titre = 90% (glass column, length = 1.2 mt., outside diameter = 6 mm, inside diameter = 4 mm; packed with Chromosorb® W.H.P. (0,15—0,18 mm) covered with silicon oil HCC—W 982; 3.8% by weight, temperature 280°C, isothermic). NMR ($CDCl_3$)v, ppm (TMS):

1.1 – 1.4 ($CH_3$) ; 1.6 – 1.8 (m, 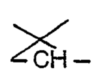CH – CO)

2.0 – 2.5 (m, 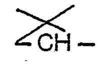CH – C = C) ; 5.0 – 5.8 (m, C<u>H</u> = CH – CH = $CCl_2$) ;

6.2 – 6.5 (m, CH – CN and CH = C<u>H</u> – CH = $CCl_2$) ;

6.9 – 7.6 (m, $CH = CCl_2$ and aromatic protons).

f) Preparation of the starting material β-trifluoromethyl-β-chloro-acrolein.

A mixture of $CF_3$—$CCl_3$ (450 g; 2.4 mols) and n-butyl-vinyl-ether (80 g; 0.8 mols) was irradiated with a mercury-vapor U.V. lamp of the Hanau HP type, model TQ 150, for 1.5 hours at 45°C. The reaction mixture was then distilled in order to remove the unconverted reactants. Thereby was obtained a residue consisting of 1,1,3-trichloro-4,4,4-trifluorobutyl-butyl-ether, chromatographically pure (yield = 89%). 0.1 mols of this compound were thereupon dripped, at room temperature, into a solution containing 0.1 mols of sodium-carbonate decahydrate in 100 ml of water.

This solution was maintained under stirring for 3 hours. Thereafter the organic phase was separated from the solution and anhydrified and then distilled at atmospheric pressure. The pure product boils at about 70°C. I.R. analysis: 3050, 1685, 1625 $cm^{-1}$; NMR ($CDCl_3$) (δ, ppm)

H
|
6.7 [d,C=C, J = 7 Hz]

10.1 [d, CHO, J = 7 Hz].

d = doublet, J = coupling constant.

The preparation was then repeated in the absence of a base, operating in the following way:

50 g (0.174 mols) of 4,4,4-trifluoro-3,3,1-trichlorobutylbutylether were introduced into a 250 ml flask fitted with a stirrer and a reflux condenser. After the addition of 150 ml of water, the content was heated at reflux temperature (temperature of the mixture = about 85°C) for 1 hour. At this point the flask was connected to a Vigreux column and the content was distilled thereby gathering the fraction that boiled between 71°C and 75°C.

After anhydrification on $Na_2SO_4$ and filtering, there were obtained 15 g of β-trifluoromethyl-β-chloroacrolein.

$g_1$) Hydrolysis of 2-acetoxymethyl-3,3-dimethyl-carbethoxy-cyclopropane (1) for obtaining 2-hydroxymethyl-3,3-dimethyl-carbethoxy-cyclopropane trans(2) and 6,6-dimethyl-3-oxa-bicyclo[3,1,0]-hexan(2)one(3)

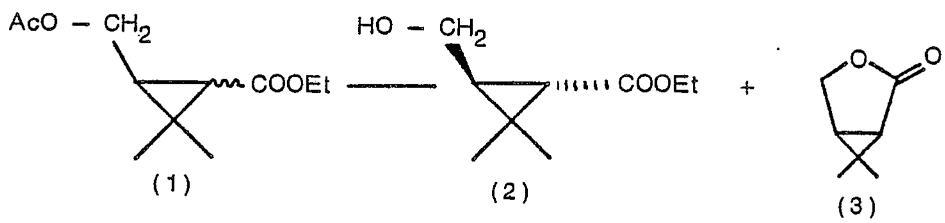

(1) (2) (3)

1 mol of (1) was dripped, at a temperature of between 0° and 10°C, into a beaker containing 500 ml of 99% ethyl alcohol and 0.25 mols of metal sodium. This mixture was then kept under stirring at room temperature overnight. Thereafter the solvent was evaporated at 15 mmHg until obtaining about 250 ml of solution. This was poured into 750 ml of water and ice and was then extracted with methylene chloride (4 times, each time with 150 ml of solvent).

The reunited organic phases were then washed with water until reaching a neutral pH, whereupon they were anhydrified and evaporated, thereby obtaining 140 g of an oil which by gas-liquid chromatographic analysis (GLC) appeared to consist for 35% of (3) and for 50% of (2) (yield about 80%). The raw product was distilled and rectified at 20 mbar.

There were obtained 0.37 mols of (3) (b.p. at 20 mbar = 112—114°C) and 0.33 mols of (2) (b.p. at 20 mbar = 132—134°C).

IR analysis of (3):

1780 $cm^{-1}$

IR analysis of (2):

3200—3600 $cm^{-1}$, 1715 $cm^{-1}$,
1380—1370 $cm^{-1}$, 1170 $cm^{-1}$, 1020 $cm^{-1}$.

$g_2$) Preparation of 2-bromomethyl-3,3-dimethyl-carbethoxy-cyclopropane trans (4) (Method C)

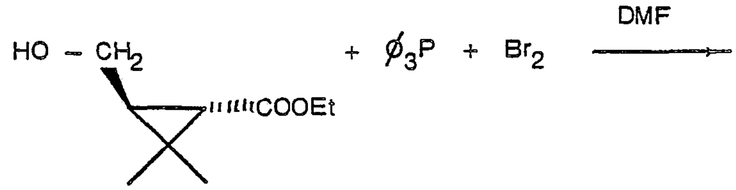


(2)

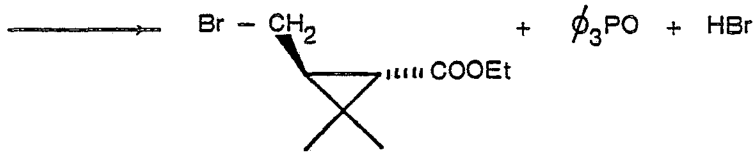


(4)

About 0.3 mols of bromine are dripped into a solution of 0.27 mols of 2-hydroxymethyl-3,3-dimethyl-carboethoxycyclopropane (1), 0.28 mols of triphenylphosphine and 300 ml of dimethylformamide, maintaining the temperature at between 50°C and 55°C (dripping time about 30 minutes).

Once the dripping has been completed, the mixture was maintained under stirring at 50°C for 5 minutes. Thereafter it was cooled down and then poured into 1.2 liters of water and ice, after which it was extracted with benzene (5 times, each time with 150 ml of solvent).

The re-united organic extracts were then washed with an aqueous sodium sulphite solution at 5% concentration, then with a 5% sodium bicarbonate solution and finally with water until attaining a neutral pH. The mixture was thereupon anhydrified on sodium sulphate and then evaporated thereby obtaining a raw product of a rubbery appearance. It was then diluted with petroleum ether, and the white solid that precipitated (70 g of triphenyl-phosphoxide equal to 0.25 mols) was filtered. After evaporation of the solvent there were obtained about 0.25 mols of (2) (yield = 90—95%). b.p. (at 0.067 mbar) = 65°—66°C.

NMR (δ, ppm, TMS):

4.1 [q, $COOCH_2$]
3.4 [dd, $CH_2Br$]
1.7—2.1 [m, H of cyclopropane]
1.2 [s, $CH_3$ geminals]
1.2 [t, $CH_3$—$CH_2O$]

(s = singlet, dd = doublet of doublet, t = triplet, q = quartet, m = multiplet).

$g_3$) Preparation of 2-bromomethyl-3,3-dimethyl-carbethoxy-cyclopropane-cis, starting from 6,6-dimethyl-3-oxa-bicyclo[3,1,0]-hexan-(2)-one. (Method C).

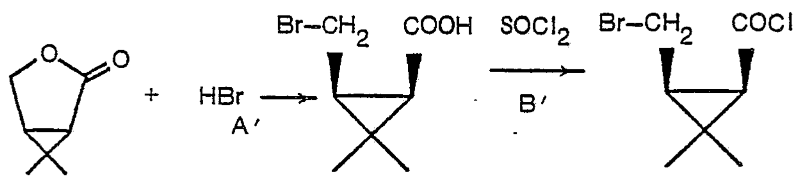


(5)

(3)

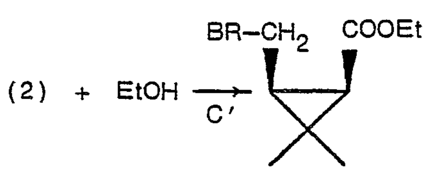


A') Into a solution of lactone (1) (0.37 mols) and of 60 cc of 99.5% ethanol, were made to bubble through 0.98 mols of gaseous hydrogen bromide. The temperature was rigorously maintained at between 0°C and 2°C. (Bubbling time = 5 hours). The solution was then kept under stirring at 0°C for another 19 hours.

Cooling the solution down, there were added 160 ml of water and ice. The resulting white precipitate was filtered. The filtrate was then washed with water until attaining a neutral pH (5 times, each time with

50 ml) and successively was washed with petroleum ether. It was then anhydrified and the petroleum ether was evaporated, thereby obtaining 0.055 mols of 2-bromomethyl-3,3-dimethyl-carbethoxycyclopropane. From the washing waters, by extraction with methylene chloride there were obtained about 3 g of the starting product. The white solid filtrate which, after drying, weighed 50 g, showed a melt point (m.p.) of 115°—117°C, and proved soluble in 5% bicarbonate.

The elemental analysis (in %) was:

theor. C = 40.60; found C = 40.71
theor. H = 5.35; found H = 5.37
theor. Br = 38.60; found Br = 38.54

B') The 2-bromomethyl-3,3-dimethyl-carboxycyclopropane obtained according to point A' (0.18 mols) was dissolved in 180 cc of chloroformium. Thereupon, by dripping, there was added the thionyl chloride (0.36 mols) cooling with water and ice so that the reaction temperature did not exceed 10°C. The reaction mixture was then reflux heated until there is no more development of hydrochloric acid (about 5 hours). It was then evaporated at reduced pressure thereby obtaining a raw oil (yield = about 95%). The raw product thus obtained partly decomposes by distillation and is then used as such in stage C). By a distillation test on a reduced quantity, there was obtained an 85% pure product (GLC) with a b.p. (at 0.2 mbar) = 50°—55°C. I.R. analysis: 1770 $cm^{-1}$.

C') 0.18 mols of the raw acyl chloride prepared according to point B, were admixed to 50 ml of anhydrous benzene. While cooling the mixture with water and ice, there were dripped into it 0.36 mols of a 99.9% ethanol.

Once the addition of the ethanol was accomplished, there were dripped in slowly, at about 0°C, 0.18 mols of pyridine. The mixture was then allowed to rest overnight at room temperature and the salt that had formed was filtered. The filtrate, after washing to a neutral pH with water, was anhydrified on sodium sulphate and then evaporated. Thereby were obtained 40 g of oil which was distilled.

B.p. (at 20 mbar) = 107°—110°C.
I.R. analysis: 1720 $cm^{-1}$.

Example 4

Preparation of the 3-phenoxy-benzyl ester of (±, trans), 2,2-dimethyl-3-(4',4'-dichlorobutadienyl)-cyclopropanecarboxylic acid (3) [compound No. II].

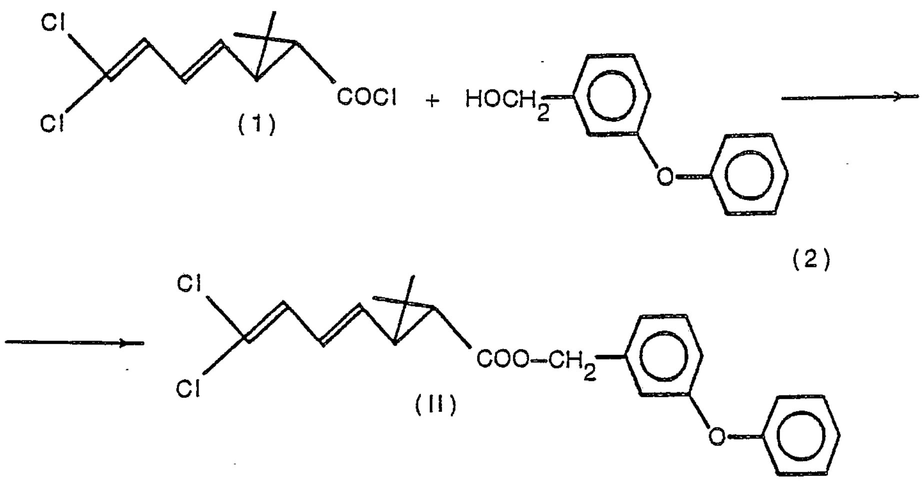
(1) (2) (II)

0.25 g of compound (1), prepared as described in example 3 part c), were diluted with 0.5 ml of anhydrous benzene. It was then cooled down to 0°C and into it there was dripped a mixture consisting of 3-phenoxy-benzyl alcohol (2) (0.1 m mols) and of 0.5 ml of anhydrous benzene. Then, still at 0°C, there was dripped into it a mixture consisting of 0.9 m mols of pyridine and 0.5 ml of anhydrous benzene. This mixture was then maintained under stirring at room temperature overnight. Thereupon it was washed with water until attaining a neutral pH, anhydrified and finally evaporated, thereby obtaining 300 mg of raw product which was then purified by preparatory chromatography.

GLC: 2 isomers, total 97% (glass column, length 2 metres, outer diameter 6 mm, inner diameter 4 mm; packed with Chromosorb® W—HP (0.15—0.18 mm) covered with 3% by weight of silicon oil OV-210; isothermic temperature = 240°C). NMR ($CDCl_3$)v, ppm (TMS):

1.1 – 1.3 ($CH_3$) ; 1.5 – 1.7 (m, $\times$CH – CO) ; 2.0 – 2.3 (m, $\times$CH – C = C) ;

5.05 (s, $CH_2$–O) ; 5 – 5.8 (m, C<u>H</u> = CH–CH = $CCl_2$) ;

6.2 – 6.5 (m, CH = C<u>H</u> – CH = $CCl_2$) ; 6.8 – 7.5 (m, CH = $CCl_2$ and aromatic protons).

Example 5

Operating in the same way as that described in example 4, there was prepared the 3-phenoxy-benzyl ester of (±)trans-3-(3′,4′,4′-trichlorobutadienyl)-2,2-dimethyl-cyclopropane-carboxylic acid [compound No. III]:

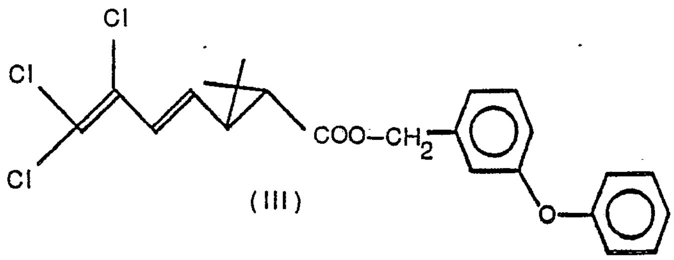

(III)

IR ($cm^{-1}$): 1715, 1620, 1580, 1480, 1250, 1210, 1160, 1110, 930, 780 and 690.
NMR ($CDCl_3$)δ, ppm (TMS):

1.2 – 1.4 ($CH_3$),

1.7 – 1.9 (m, $\times$CH – CO),

2.1 – 2.4 (m, $\times$CH – C = C),

5.1 (s, $CH_2O$),

5.5 – 6.2 (m, C<u>H</u> = CH – CCl = $CCl_2$)

6.9 – 7.6 (m, CH = C<u>H</u> – CCl and aromatic protons).

Example 6

Operating in the same way as that described in example 3, part e), there was prepared α-cyano-3-phenoxy-benzyl ester of the (±)-trans-3-(5′,5′,5′-trifluoro-4′-chloro-penta-1′,3′-dienyl)-2,2-dimethyl-cyclopropanecarboxylic acid [compound No. IV] of the formula:

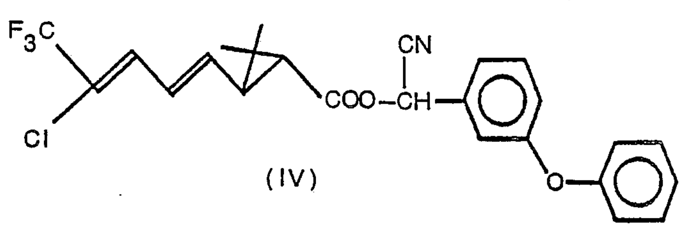

(IV)

IR ($cm^{-1}$): 1725 (ν C=O); 1630 (ν CH=CH).

Example 7

a) Preparation of 3-(E,Z-4′,4′-dichloro-2′-bromo-butadienyl)-2,2-dimethylcarbethoxycyclopropane(trans):

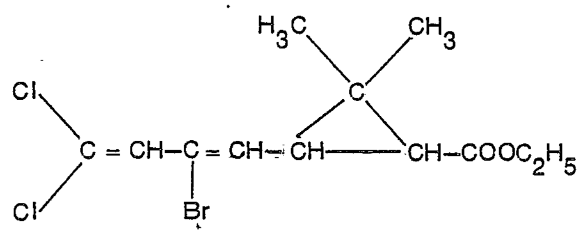

The compound was prepared from the compound of example 3 part b), [3-(4',4'-dichlorobutadienyl)-2,2-dimethyl-carbethoxycyclopropane] by bromination and dehydrobromination according to conventional methods (scheme 1c).

IR: 1730 $cm^{-1}$ (ν C=O); 1595 $cm^{-1}$ (ν C=C).

[an alternative synthesis is reported in Example 37].

b) Preparation of the chloride of 3-(E,Z-4',4'-dichloro-2'-bromo-butadienyl)-2,2-dimethyl-cyclopropanecarboxylic acid (trans):

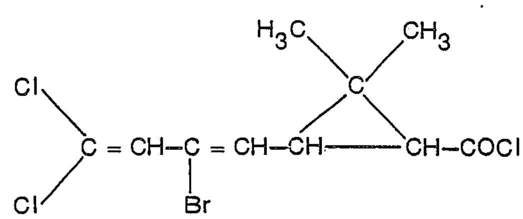

The compound has been prepared in an analogous way to that described in example 3 part c) + d), starting from the ethyl ester obtained according to part a).

IR: 1775 $cm^{-1}$ (ν C=O), 1595 $cm^{-1}$ (ν C=C).

c) Preparation of 3-phenoxy-benzyl ester of 3-(E,Z-4',4'-ro-2'-bromo-butadienyl)-2,2-dimethyl-cyclopropanecarboxylic acid (trans) [compound No. V]:

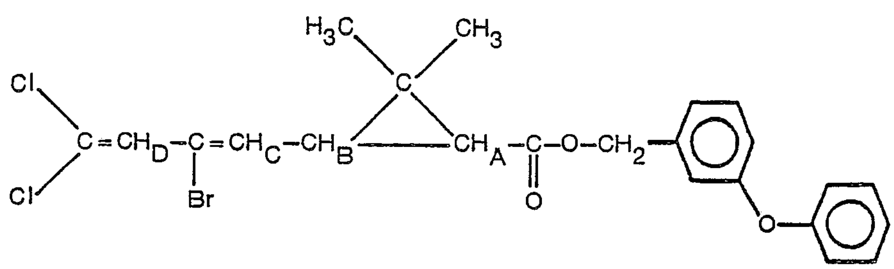

(V)

The compound was prepared using the same method as that described in example 4, starting from the above obtained acylchloride and from 3-phenoxy-benzyl alcohol.

IR: 1730 $cm^{-1}$ (ν C=O); 1590 $cm^{-1}$ (expanded band, ν C=C + aromatics);

NMR ($CDCl_3$, TMS):

δ(ppm): 1.17 (6H, geminal methyls)
1.28
1.65 (d, 1H, $H_A$)
2.02 (dd, 1H, $H_B$)
5.10 (s, 2H, $CH_2$)
5.63—6.15 (d, d, 1H, $H_C$)
6.40 (s, 1H, $H_D$)
6.75—7.55 (m, 9H, aromatic protons)
$^JH_A—H_B$=6.0 Hz
$^JH_B—H_C$ = 10.0 Hz

(s = singlet, d = doublet, dd = doublet of doublet, m = multiplet, J = coupling constant).

Example 8

Preparation of the α - cyano - 3 - phenoxy - benzyl ester of the 3 - (4',4' - dichloro - 2 - bromo - butadienyl) - 2,2 - dimethyl - cyclopropanecarboxylic acid(trans) [compound No. VI].

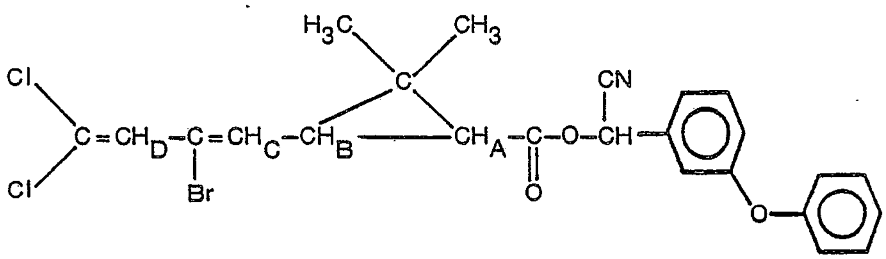
(VI)

The compound was prepared using the same method as that described in example 3 part e), starting from the acyl-chloride of example 7 part b); and from α - cyano - 3 - phenoxy - benzyl alcohol.
IR: 1740 $cm^{-1}$ (ν C=O), 1590 $cm^{-1}$ (expanded band, ν C=C + aromatics);
NMR ($CDCl_3$, TMS):
δ (ppm): 1.0—1.45 (m, 6H, geminal methyls)
1.6—2.2 (m, 1H, $H_A$)
1.9—2.2 (m, 1H, $H_B$)
5.6—6.1 (d, d, 1H, $H_C$)
6.3—6.45 (m, 2H, $H_D$ + CH—CN)
6.85—7.55 (m, 9H, aromatic protons)
(d = doublet; m = multiplet).

Example 9

Preparation of the α - cyano - 3 - phenoxy - benzyl ester of the 3 - (E,Z-2',4',4' - trichloro - butadienyl) - 2,2 - dimethyl - cyclopropanecarboxylic acid(trans) [compound No. VII].

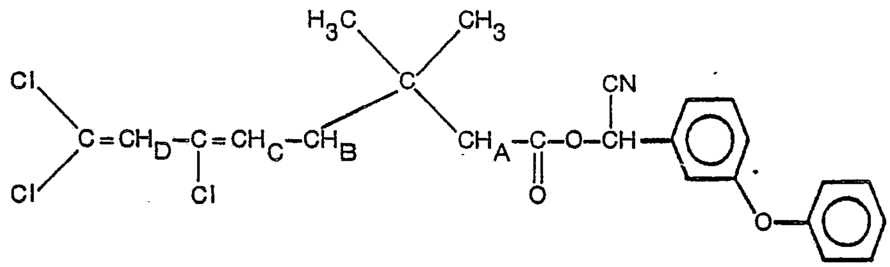
(VII)

The compound was prepared starting from the ethyl ester of the 3-(E,Z-2',4',4'-trichloro-butadienyl)-2,2-dimethyl-cyclopropanecarboxylic acid (Compound B, example 2 b) which was converted to the corresponding acyl-chloride, operating in a similar way to that described in example 3, part c + d, and then esterified with α-cyano-3-phenoxy-benzyl alcohol operating in a similar way to that described in example 16.
NMR: ($CDCl_3$, TMS)
δ (ppm): 1.05—1.45 (m, 6H, geminal methyls)
1.57—1.80 (m, 1H, $H_A$)
1.80—2.80 (m, 1H, $H_B$)
5.59—5.81 (d, d, $H_C$, E + Z)
6.35 (2H, $H_D$ + CH—CN)
6.87—7.65 (m, 9H, aromatic protons)
(d = doublet; m = multiplet).

Example 10

a) Preparation of the ethyl ester of 3-(E,Z-4'-chloro-1',3'-pentadienyl)-2,2-dimethyl-cyclopropanecarboxylic acid:

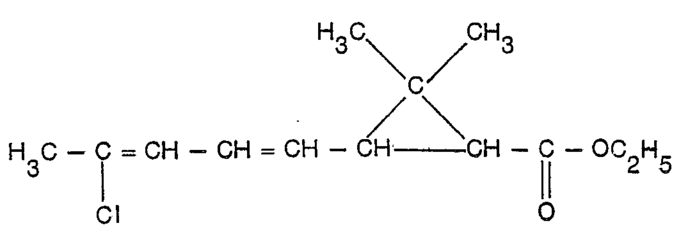

The compound was prepared according to the same procedures that have been described in example 3 part b, starting from [(2-carbethoxy-3,3-dimethyl)-cyclopropyl]-methyl-triphenylphosphoniumbromide and from β-methyl-β-chloro-acrolein

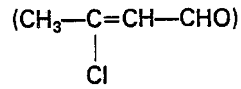

IR spectrum: meaningfull bands at: 1720, 1620, 1180 and 965 $cm^{-1}$.
[an alternative synthesis is reported in Example 13].

b) Preparation of the chloride of 3-(E,Z-4′-chloro-1′,3′-pentadienyl)-2,2-dimethyl-cyclopropanecarboxylic acid:

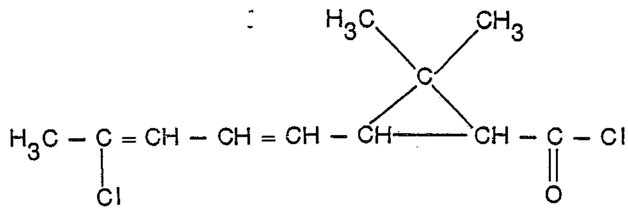

The above prepared ethyl ester was subjected to an alkaline hydrolysis thereby obtaining the corresponding carboxylic acid [IR: 1700 $cm^{-1}$ (ν C=O)] and this latter, according to the methods described in example 3 part c), was treated with thionyl chloride.

IR of acyl-chloride: 1780 $cm^{-1}$ (ν C=O)

c) Preparation of α - cyano - 3 - phenoxy - benzyl ester of the 3 - (E,Z-4′ - chloro - 1′,3′ - pentadienyl) - 2,2 - dimethyl - cyclopropanecarboxylic acid [Compound No. VIII]:

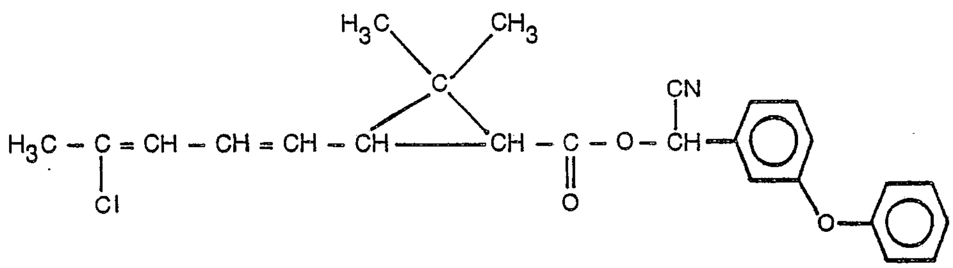

(VIII)

The compound was prepared by operating in an analogous way as that described in example 3 part e), starting from acyl-chloride of part b) and from α-cyano-3-phenoxy-benzyl alcohol. The product was obtained in the form of a mixture of cis-trans isomers on the cyclopropane ring, in a ratio of about 1:9.

IR: 1720 $cm^{-1}$ (ν C=O).

Example 11 (Method D):

a) Preparation of the diethylester of 3,3-dichloro-2-propenephosphonic acid (reaction 20):

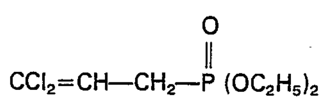

1 Into a 250 ml flask, fitted with a magnetic stirrer and a reflux condenser, there were introduced:

83 g (0.05 mols) of triethylphosphite[$P(OC_2H_5)_3$]

73 g (0.5 mols) of 1,1,3-trichloropropene $Cl_2C{=}CH{-}CH_2Cl$.

The reaction mixture was stirred for 5 hours at 160°C. It was then allowed to cool down to room temperature, whereafter it was distilled at reduced pressure thereby gathering the fraction with boiling point comprised between 145° and 153°C at a pressure of 24 mbar (104.7 g).

The fraction thus gathered proved to consist of the product desired, having a purity of 96.6% (gas chromatographic titre GLC).

IR (infrared spectroscopy), ($cm^{-1}$): 1620 (ν C=C); 1260 (ν P=O); 1025, 965, 920.

$^1H$ NMR (nuclear magnetic resonance): δ, ppm ($CDCl_3$, TMS)

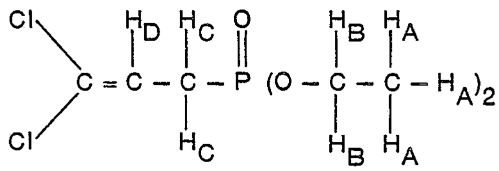

1.1—1.5 (t, 6H, $CH_3$)
2.4—3.0 (dd, 2H, $CH_2$—P)
3.8—4.4 (m, 4H,$OCH_2$)
5.7—6.2 (m, 1H, $CCl_2$=CH)
$^{J}H_A—H_B$ = 7 Hz
$^{J}H_C—P$ = 22 Hz
$^{J}H_C—H_D$ = 8 Hz

(t = triplet; dd = doublet of doublet, m = multiplet; J = coupling constant).

b) Preparation of the diethyl ester of 3,3-dichloro-1-bromo-2-propene-phosphonic acid (reaction 21):

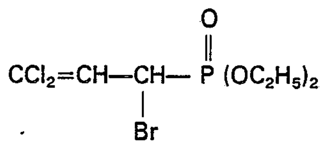

Into a 1 litre flask, fitted with a mechanical stirrer and with a reflux condenser, closed by a calcium chloride valve, there were introduced: 125 g (0.5 mols) of diethylester of 3,3-dichloro-2-propenephosphonic acid (obtained as described above). 100 g (0.56 mols) of N-bromosuccinimide, 500 ml of carbon tetrachloride, 1.0 g of azo-bis-isobutyronitrile (A.B.N.).

This mixture was then reflux heated, under constant stirring for 2 hours, after which there were admixed further 1.0 g of A.B.N. and refluxing was carried on under stirring for another 2 hours. After cooling down to 0°C, the mixture was then filtered in order to remove the succinimide and from the filtrate was then evaporated the solvent.

The residue was diluted in 500 ml of petroleum ether and then was cooled down to 0°C. Once again there precipitated a small quantity of succinimide which was removed by filtering.

From this filtrate, after evaporation of the solvent, there was obtained a residue which was purified by chromatography on a silica gel column (eluent: petroleum ether-$CH_2Cl_2$-ethyl acetate in the ratio of 10:6:4).

Thereby were obtained 135 g of the desired product. The product analysis showed:

IR: in agreement with the assigned structure;

Elemental analysis:
C, in %: theoretical: 25.75 — found: 25.43
H, in %: theoretical: 3.71 — found: 3.64
Cl, in %: theoretical: 21.75 — found: 21.77
Br, in %: theoretical 24.52 — found: 25.39
P, in %: theoretical: 9.50 — found: 9.22

NMRδ, ppm ($CDCl_3$, TMS)

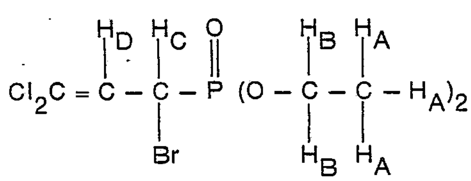

1.35 (t, 6H, $CH_3$)
4.22 (dq, 4H, O—$CH_2$)
4.68 (dd, 1H, CHBr)
6.16 (dd, 1H, =CH)
$J_{H_A-H_B}$ = 7 Hz
$J_{H_B-P}$ = 8.5 Hz
$J_{H_C-H_D}$ = 11.5 Hz
$J_{H_C-P}$ = 11.5 Hz
$J_{H_D-P}$ = 8 Hz.

(t = triplet; dq = doublet of quartet; dd = doublet of doublet; J = coupling constant).

c) Preparation of ethyl ester of trans-2,2-dimethyl-3-(2-bromo-4,4-dichlorobutadienyl)-cyclopropanecarboxylic acid.

Starting from: 8.5 g of ethyl ester of trans-caronaldehyde and 16.5 g of ethyl ester of 1-bromo-3,3-dichloro-2-propene-phosphonic acid (obtained as described above), and by operating in a way similar to that described in example 12 part d), there were obtained 9.5 grams of the desired product (in this case it is not necessary a further purification by distillation).

IR ($cm^{-1}$) 1730 (ν C=O), 1595 (ν C=C)

NMRδ, ppm ($CDCl_3$, TMS):

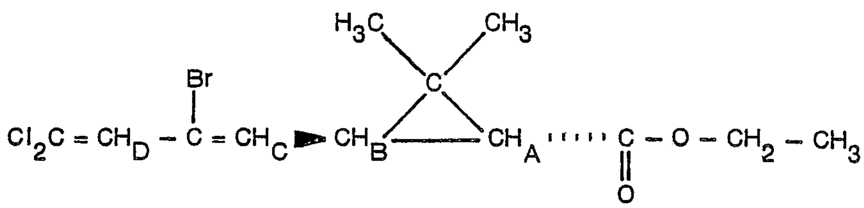

1.26 (m, 9H, methyl groups)
1.63 (d, 1H, $H_A$)
1.98 (dd, 1H, $H_B$)
4.15 (q, 2H, $CH_2$)
5.7—6.15 (dd, 1H, $H_C$)
6.37 (s, 1H, $H_D$)
$J_{CH_3-CH_2} = 7.0$ Hz
$J_{H_B-H_A} = 6.0$ Hz
$J_{H_B-H_C} = 9.0$ Hz

(s = singlet; d = doublet; dd = doublet of doublet; q = quartet; m = multiplet; J = coupling constant).

## Example 12

a) Preparation of the diethyl ester of 2,3,3,3-tetrachloropropane-phosphonate acid (Reaction 25)

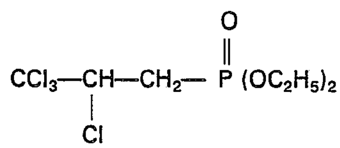

Into a 500 ml flask, fitted with a mechanical stirrer, a thermometer and a reflux condenser connected with a $H_2SO_4$ valve and with one column for the separation of the gases, there were introduced: 150 ml of benzene, 24.7 g of diethyl ester of 3,3-dichloro-2-propene-phosphonic acid (prepared as described in example 11 part a).

This mixture was thereupon irradiated under stirring, with an Osram "Sonne"® 300 Watt lamp, for 30 minutes, during which the internal (inside) temperature rose to 45°C showing a development of gas.

After cooling down to room temperature, the solvent was evaporated at reduced pressure, thereby obtaining a residue (32 grams) consisting of the desired product.

Elemental analysis:
Cl, in %: theoretical: 44.60 — found: 42.63
P, in %: theoretical 9.79 — found: 9.80
IR ($cm^{-1}$): 1265, 1230, 1170, 1025, 970.

b) Preparation of the diethyl ester of 3,3,3-trichloro-1-propene-phosphonic acid: (Reaction 26)

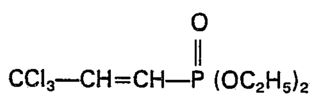

Into a beaker of 300 ml holding capacity, fitted with a magnetical stirrer and a reflux condenser, closed by a calcium chloride valve, there were introduced 16 g of diethyl ester of 2,3,3,3-tetrachloropropane-phosphonic acid (prepared as described above): 50 ml of n-hexane; 6 ml of diethylamine.

This mixture was then subjected to stirring at room temperature for 3 hours whereafter it was allowed to rest overnight. After filtering the chlorohydrate that had formed, the filtrate was washed with 10 ml of HCl 2N, then with water until obtaining a neutral pH. After anhydration with anhydrous $Na_2SO_4$ and subsequent evaporation of the solvent at reduced pressure, there was obtained a residue (12.4 g) consisting of the desired product (b.p. = 84°—85°C at 0.105 mbar).

IR ($cm^{-1}$): 1635; 1260; 1210; 1165; 1020; 970;
NMRδ, ppm, ($CDCl_3$, TMS):

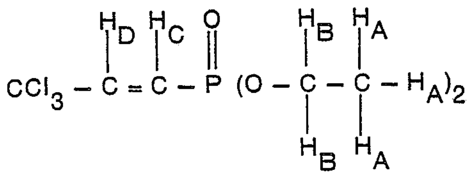

1.40 (t, 6H, $CH_3$)
4.18 (dd, 4H, $CH_2$)
6.32 (dd, 1H, $H_C$)
7.10 (dd, 1H, $H_D$)
$J_{H_A-H_B} = 7$ Hz
$J_{H_B-P} = 9$ Hz
$J_{H_C-H_D} = 16$ Hz
$J_{H_C-P} = 14.5$ Hz
$J_{H_D-P} = 20$ Hz

(t = triplet; dq = doublet of quartet; dd = doublet of doublet; J = coupling constant).

c) Preparation of the diethyl ester of 1,3,3-trichloro-2-propene-phosphonic acid (Reaction 27):

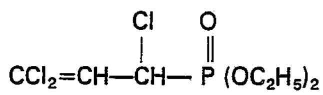

Into a 100 ml beaker, fitted with a magnetical stirrer and with a reflux condenser closed by a calcium chloride valve, there were introduced: 2.8 g of diethyl ester of 3,3,3-trichloro-1-propene-phosphonic acid (obtained as described above), 1 g of $CuCl_2.2H_2O$.

This mixture was thereupon stirred for 1 hour and 15 minutes at 110°C. After cooling down to room temperature, it was additioned with 100 ml of ethyl ether and then filtered.

The filtrate was then washed with water until achieving the full disappearance of the green colour, after which it was anhydrified on anhydrous $Na_2SO_4$. The solvent was then evaporated under reduced pressure, thereby obtaining 25 g of the desired product (b.p. = 95°—96°C/ 0.105 mbar).

Elemental analysis:
C, in %: theoretical = 29.87 — found = 29.21
H, in %: theoretical = 4.3 — found = 4.01
P, in %: theoretical = 11.0 — found = 10.91
Cl, in %: theoretical = 37.78 — found = 36.90
IR ($cm^{-1}$): 1615, 1270, 1025, 980, 920
NMRδ, ppm ($CDCl_3$ TMS)

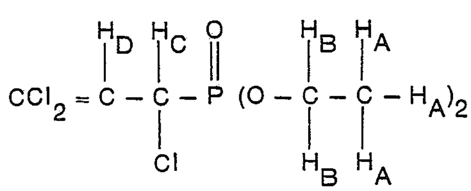

1.39 (t, 6H, $CH_3$)
3.85—4.50 (m, 4H, $CH_2$)
4.7 (dd, 1H, $H_C$)
6.08 (dd, 1H, $H_D$)
$J_{H_A-H_B} = 5.5$ Hz
$J_{H_C-H_D} = 10$ Hz
$J_{H_C-P} = 13$ Hz
$J_{H_D-P} = 6$ Hz

(t = triplet, m = multiplet, dd = doublet of doublet, J = coupling constant).

d) Preparation of ethyl ester of the trans-2,2-dimethyl-3-(2,4,4-trichloro-butadienyl)-cyclopropanecarboxylic acid (Reaction 18).

Into a 250 ml flask, fitted with a mechanical stirrer, a reflux condenser and a dripping funnel anhydrous

and kept under a nitrogen atmosphere, there were introduced 2.5 g of a suspension of sodium hydride (50%) in paraffine oil. This suspension was washed with n-hexane (3 × 20 ml) whereafter it was additioned with 100 ml of anhydrous tetrahydrofurane (THF).

This mixture was thereupon cooled down to −30 °C and then additioned with 0.2 g of imidazol and 8.5 g of ethyl ester of trans-caronaldehyde.

The mixture was then subjected to stirring and was additioned slowly over 30 minutes, by dripping, with a solution of diethyl ester of 1,3,3-trichloro-2-propene-phosphonic acid (obtained as described above) in 30 ml of anhydrous THF.

During the admixture, the temperature rose up to −10°C. The mixture was allowed to spontaneously warm up to room temperature continuing to stirrer for 2 hours and 30 minutes.

The reaction mixture was then poured into 100 g of water and ice; whereafter it was extracted with diethyl ether (3 × 100 ml).

The reunited organic phases were then washed with water up to a neutral pH, thereupon anhydrified on anhydrous $Na_2SO_4$. The solvent was evaporated at reduced pressure, thereby obtaining a residue (16 g) which was purified by chromatography on a silica gel column (eluent n.hexane/ethyl acetate = 8:2).

There were obtained 13 grams of a product which was further purified by distillation and a fraction was gathered with b.p. = 118°—122°C at a pressure of 0.13 mbar, and which consisted of the desired product (9.8 g).

IR ($cm^{-1}$) 1725, 1602

$^1H$ NMRδ, ppm ($CDCl_3$, TMS)

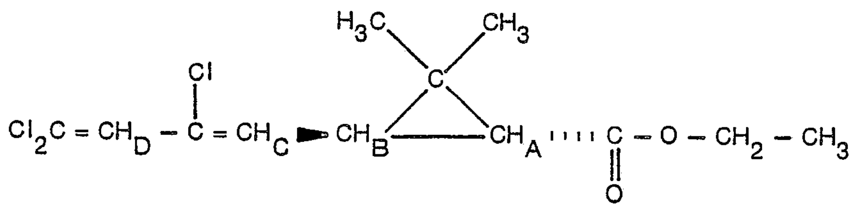

1.05—1.55 (m, 9H, methyl groups)
1.63 (d, 1H, $H_A$)
1.98 (dd, 1H, $H_B$)
5.62—5.85 (dd, 1H, $H_C$, E + Z)
6.48 (s, 1H, $H_D$)
4.13 (q, 2H, $OCH_2$)
$J_{CH_3-CH_2}$ = 7 Hz
$J_{H_A-H_B}$ = 5.5 Hz
$J_{H_B-H_C}$ = 9.5 Hz

(s = singlet; d = doublet; dd = doublet of doublet; q = quartet; m = multiplet; J = coupling constant).

Example 13:

a) Preparation of diethyl ester of 3-chloro-2-butene-phosphonic acid. (Reaction 20)

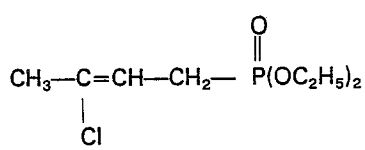

Into a 100 ml flask, fitted with a magnetical stirrer and a reflux condenser, there were introduced: 33.2 g (35 ml, 0.2 mols) of triethylphosphite, 25 g (22 ml; 0.2 mols) of 1,3-dichoro-2-butene

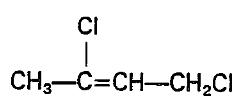

The reaction mixture was thereupon subjected to stirring for 4 hours at 150°C, whereafter it was allowed to cool down to room temperature. Then it was distilled at reduced pressure, gathering the fraction with a b.p. of 145°—146°C at 15 mm Hg (37 g) and consisting of the desired product.

NMRδ, ppm ($CDCl_3$, TMS)

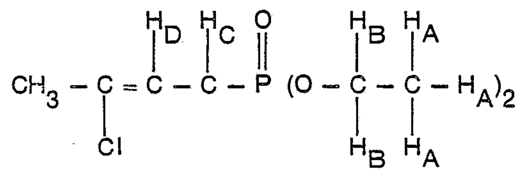

1.1—1.5 (t, 6H, $H_A$)
2.0—2.2 (d, 3H, $CH_3$—C=)
2.4—3.0 (dd, 2H, $H_C$)
4.0—4.3 (dq, 4H, $H_B$)
5.4—5.7 (m, 1H, $H_D$)
$J_{H_A-H_B}$ = 8 Hz
$J_{H_B-P}$ = 8 Hz
$J_{H_D-H_C}$ = 8 Hz
$J_{H_C-P}$ = 20 Hz

(d = doublet; t = triplet; dd = doublet of doublet; dq = doublet of quadruplet; m = multiplet, J = coupling constant).

b) Preparation of ethyl ester of cis,trans - 2,2 - dimethyl - 3 - (4 - chloro - 1,3 - pentadienyl) - cyclopropanecarboxylic acid.

Starting from: 7.2 g (0.04 mols) of ethyl ester of caronaldehyde (cis-trans mixture in a ratio of 1:8.5) and from 0.037 mols of diethyl ester of 3-chloro-2-butenphosphonic acid (prepared as described above), and by operating in a way similar to that described in example 34, there were obtained 7.6 g of a raw substance which was then purified by chromatography on silica gel (eluent n.hexane/ethyl acetate in a ratio, of 85:15) obtaining 3.7 g of the desired product (a yellowish oil).

IR ($cm^{-1}$) 1720, 1620, 1180, 965

NMRδ, ppm ($CDCl_3$, TMS)

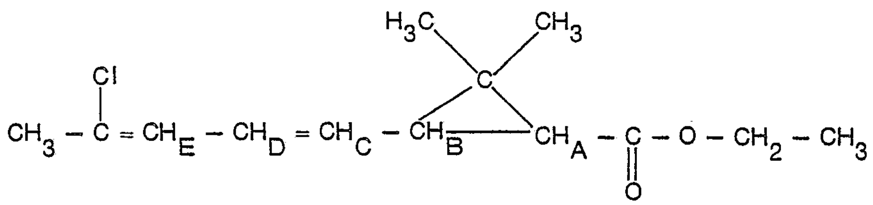

1.1—1.4 (9H, $CH_3$—$CH_2$ and geminal methyls)
1.5—1.7 (1H, $H_A$)
2.1—2.4 (4H, $CH_3$—CCl and $H_B$)
3.9—4.3 (2H, $CH_2$)
5.0—6.8 (3H, $H_C$, $H_D$ and $H_E$)

Example 14

Preparation of ethyl ester of 2,2-dimethyl-3-(4,4-dichlorobutadienyl)cyclopropanecarboxylic acid (Reaction 18):

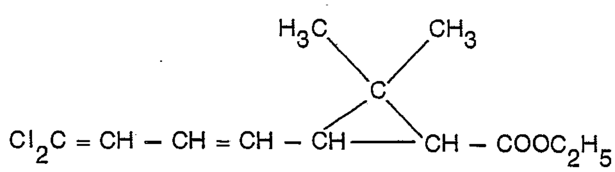

Into a 500 ml flask, anhydrous and maintained under a nitrogen atmosphere, there were introduced 12.5 g of a suspension of about 50% of sodium hydride (0.26 mols) in paraffine oil. This suspension was then washed with anhydrous petroleum ether (2 × 50 ml) and to it were then admixed 200 ml of anhydrous dimethoxyethane.

To this mixture, kept under stirring at 5°—10°C under a nitrogen atmosphere, there was slowly dripped, in a period of 2 hours, a mixture consisting of: 49.5 g (0.2 mols) of diethyl ester of 3,3-dichloro-2-propene-phosphonic acid (obtained as described in example 11 part a), 0.24 mols of ethyl ester of the caronic aldehyde (cis-trans mixture in a ratio of 58:42).

Once the admixture had been accomplished, the mixture was allowed to spontaneously heat up to room temperature while keeping it under constant stirring for 1 hour.

The reaction mixture was then cooled down to 10°C and to it were then admixed dropwise 5 ml of ethyl alcohol and then 150 ml of a 1% hydrochloric acid. The mixture was then extracted with ethyl ether

(2 × 100 ml). The reunited organic phases were then washed with water until reaching a neutral pH, anhydrified on anhydrous sodium sulphate and the solvent eliminated under reduced pressure.

Thereby were obtained 76.2 g of a raw product (dark oil) which was distilled under reduced pressure thereby gathering the fraction with b.p. of 105°—109°C at 0.067 mbar (19 grams) consisting of the desired product (mixture of geometrical isomers) with a purity degree of 93% (GLC).

Elemental analysis:
C, in %: theoretical = 54.77 — found = 53.77
H, in %: theoretical = 6.13 — found = 6.18
Cl, in %: theoretical = 26.95 — found = 25.65
The IR and NMR spectroscopic data were consistent with the assigned structure.
The thus prepared compound may be used in the process of example 3 part e) or example 4.

Example 15:

a) Preparation of O-ethyl-O-(2'-chloroethyl)-3,3-dichloro-2-propene-phosphonate. (Reaction 21).

Into a 50 ml flask, fitted with a magnetical stirrer, a thermometer, a reflux condenser and connected with a $H_2SO_4$ valve, under a nitrogen atmosphere and at room temperature, there loaded: 14.5 g (0,1 mols) of 1,1,3-trichloro-propene, 13.5 g (0.1 mols) of 2-ethoxy-1,3,2-dioxaphospholane of the formula:

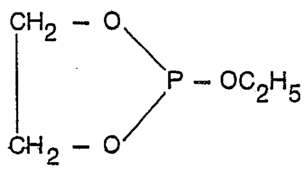

The reaction mixture was then heated up to 140°C and then, in 2 hours and 30 minutes, the temperature was brought up to 165°C. The reaction mixture was thereupon allowed to cool down to room temperature and was then distilled at reduced pressure, gathering the fraction that distilled at between 120° and 130°C at a pressure of 0.27 mbar. The gathered fraction (15.3 g) proved to consist of the desired product showing a purity of 92% (GLC).

IR ($cm^{-1}$): 1620 (ν C=C), 1260 (ν P=O), 1025, 970, 920 (ν P—O—C)
NMR ($CDCl_3$, TMS)

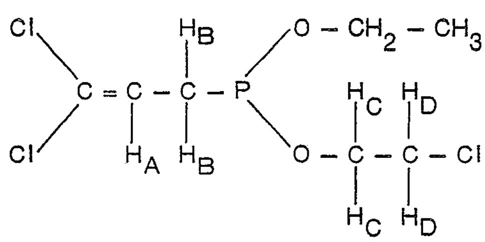

δ (ppm):
1.2—1.5 (t, 3H, $CH_3$)
2.5—3.1 (dd, 2H, $H_B$)
3.6—3.8 (t, 2H, $H_D$)
4.0—4.5 (m, 4H, $CH_2$—$CH_3$ + $H_C$)
5.7—6.15 (m, 1H, $H_A$)
$J_{CH_3-CH_2}$ = 7 Hz
$J_{H_B-P}$ = 22 Hz
$H_{H_B-H_A}$ = 8 Hz
$J_{H_D-H_C}$ = 5 Hz

(dd = doublet of doublet; t = triplet; m = multiplet; J = coupling constant).

b) Preparation of ethyl ester of 2,2-dimethyl-3-(4,4-dichlorobutadienyl)-cyclopropanecarboxylic acid. (Reaction 18)

Into a 100 ml flask, maintained in a nitrogen atmosphere, there were introduced 2 g of a 55% suspension of sodium hydride (NaH) in vaselin oil. The oil was then extracted by repeated washings (3 × 25 ml) with n.hexane, and there were then additioned 20 ml of tetrahydrofurane (THF). To this suspension, kept under stirring at 10°C, there were then slowly admixed in 1.5 hours a mixture consisting of: 3.4 g (0.02 mols) of caronic aldehyde (a cis-trans mixture), 7.5 g (0.025 mols) of the phosphonate of part a), 5 ml of THF.

Once the additions had been accomplished, the reaction mixture was subjected to stirring for further 30 minutes at 10°C. Thereupon the reaction mixture was poured slowly into 100 ml of water and ice, after which it was extracted with diethylether (3 × 50 ml).

The reunited extracts were then washed with cold water until attaining a neutral pH (3 × 50 ml), they were then anhydrified on anhydrous $CaCl_2$ and the solvent was then removed by evaporation at reduced pressure.

Thereby were obtained 6 grams of a raw oil which was chromatographied on silica gel (eluent n-hexane/ethyl ether in a ratio of 95:5).

There were obtained 4 grams of the desired product in the form of mixture of geometric isomers having a purity degree ≥95% (GLC).

The elemental analysis, the IR and NMR values were consistent with the assigned structure.

## Example 16

Preparation of $CF_3—CCl_2—CH_2—CCl_3$.

Into a Pyrex glass vial were introduced: 10 g of 1,1,1-trichlorotrifluoroethane (0.05 mols), 5 g of 1,1-dichloroethylene (0.05 mols), 0.2 g of diethylamine-chlorohydrate, 0.2 g of $CuCl_2.2H_2O$ and 8 ml of acetonitrile.

After sealing on a flame, the vial was heated for 24 hours at 120°C. Thereupon it was cooled down and opened and the content was diluted with 50 ml of methylene chloride and then washed with water (3 × 100 ml), after which it was anhydrified on sodium sulphate and subjected to distillation in order to remove the solvent and the more volatile fractions consisting mainly of unreacted trichlorotrifluoroethane.

As a residue there were gathered 5 g of a fluid yellowish liquid, mainly consisting of 1,1,1,3,3-pentachloro-4,4,4-trifluorobutane.

NMR (nuclear magnetic resonance) δ = 3.8 ppm ($CH_2$) in $CDCl_3$.

Mass spectrometry:

($C_4H_2Cl_5F_3$)
247 ($M^+$—Cl)
211 (247—HCl)
151 ($CF_3—CCl_2^+$)
117 ($CCl_3^+$)
69 ($CF_3^+$)

b) Preparation of ethyl 3,3-dimethyl-4,6,6,8,8-pentachloro-9,9,9-trifluoro-nonanoate: (Method B):

$$CF_3—CCl_2—CH_2—CCl_2—CH_2—CHCl—C(CH_3)_2—CH_2—COOC_2H_5.$$

Into a 50 ml flask fitted with a stirrer and a reflux condenser, connected by its upper end to a $CaCl_2$ valve, there were introduced: 5.7 g of $CF_3—CCl_2—CH_2—CCl_3$ (0.02 mols) (obtained as described above), 3.1 g of ethyl 3,3-dimethylpent-4-enoate (0.02 mols), and about half of a solution obtained by dissolving 0.5 ml of $Fe(CO)_5$ in 5 ml of isopropyl alcohol.

This reaction mixture was thereupon heated up under a slight refluxing at about 100°C and additioned, in 3 hours, with the remaining iron-pentacarbonyl [$Fe(CO)_5$] solution. After cooling down, the content was then diluted with 50 ml of methylene chloride, then washed with water, anhydrified and finally concentrated under vacuum in order to give 8.5 g of a raw product which was then distilled under vacuum gathering thereby the fraction boiling at 115°—118°C (0.2 mbar) and which consisted of the desired product.

## Example 17

Operating in the same way as that described in example 3, part c) and d) the following compounds were prepared from the corresponding starting materials:

— Chloride of 3-(3′,4′,4′-trichlorobutadienyl)-2,2-dimethylcyclopropanecarboxylic acid (trans) of formula:

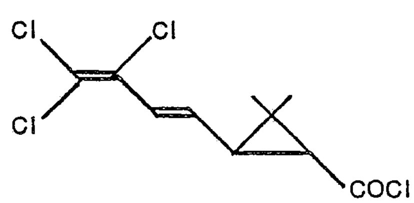

IR ($cm^{-1}$):
1765 (ν C=O)
1585 (ν $CCl_2$= CCl)
1620 (ν CH=CH);

— Chloride of 3-(5′,5′,5′-trifluoro-4′-chloro-penta-1′,3′-dienyl)-2,2-dimethyl-cyclopropanecarboxylic acid of formula:

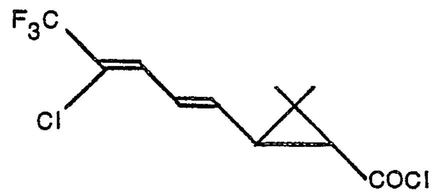

IR ($cm^{-1}$):
1765 (ν C=O)
1580 (ν $CF_2$—CCl=CH)
1630 (ν CH=CH).

These compounds may be converted into corresponding final products according the process described in example 3 part e).

Example 18

Insecticide activity of the compounds of the invention. The compounds of this invention were tested on larvae and adults of the following phytophagouses, according to the following methodologies.

A) *Biological activity on Macrosiphum euphorbiae (aphides):*

Pot-grown potato plants were infested with adult females of aphides and, after a few hours, were besprinkled with a hydroacetonic dispersion of the products under examination.

The mortality percentage was determined 24 hours after treatment (mortality of the aphides on untreated plants was equal to 0).

B. *Biological activity on Pieris brassicae (Lepidoptera):*

Cut cauliflower leaves were submitted to besprinkling with a hydroacetonic dispersion of the products under examination.

After drying, the leaves were infested with 5-days old larvae. The mortality percentage of said larvae (mortality on untreated leaves = O) was determined 48 hours after treatment.

C) *Biological activity on Leptinotarsa decemlineata (Coleoptera).*

Small pot-grown potato plants were infested with 4-days old larvae and subsequently subjected to besprinkling with a hydroacetonic dispersion of the products under examination. The mortality percentage (untreated plants, mortality = 0) was determined 48 hours after the treatment.

D. *Biological activity on Musca domestica (Diptera):*

4-days old adults were treated, by topical application with a microsyringe, with an acetonic solution of the products under examination.

The mortality percentage (mortality of the insects treated only with acetone = 0) was determined 24 hours after treatment.

E) *Biological activity on Blatta orientalis (Orthoptera):*

The bottom and walls of crystallizers of glass were uniformly treated with an acetonic solution of the products under examination.

After evaporation of the solvent, in each crystallizer there were introduced 80—100 days old neanides, thereupon closing the crystallizers with a metal net cover. 24 hours after treatment the insects were transferred in similar, untreated crystallizers and suitably fed. The mortality percentage (mortality of untreated insects = 0) was determined 48 hours after the start of the treatment.

F) *Biological activity on Tetranychus urticae adults (Acari):*

Bean leaf discs were infested with adult acari and successively besprinkled with a hydroacetonic dispersion of the products under examination.

The mortality percentage was determined 24 hours after treatment (mortality of the acari on untreated foliar discs = O).

G) *Biological activity on Spodoptera littoralis:*

Cut tobacco leaves were besprinkled with a hydroacetonic dispersion of the products under examination.

After drying, the leaves were infested with 5-days old larvae. The percentage of mortality of the larvae was determined 48 hours after treatment.

(The mortality of the larvae on untreated leaves = 0).

H) *Biological activity on Tetranychus urticae eggs (Acari):*

Bean leaf discs, previously infested with acari eggs, were treated by besprinkling with a hydroacetonic dispersion of the products under examination. The percentage of unhatched eggs (equivalent to the mortality percentage) was evaluated, 6 days after treatment, in comparison with the percentage of unhatched eggs on the untreated foliar discs.

The biological activity data of some representative compounds have been recorded on the following Table 1 and are expressed as a mortality percentage at the indicated dose.

TABLE 1

Biological activity of compounds of the invention expressed as percentage of mortality of the pests at the indicated doses

| Compound n° (Example n°) | Macrosiphum e. (0.1‰) | Pieris b. (0.1‰) | Leptinotarsa d. (0.1‰) | Spodoptera l. (0.1‰) | Musca d. (0.1γ/ins.) | Tetranichus u. (adults) (0.1‰) | Blatta o. (0.1‰) |
|---|---|---|---|---|---|---|---|
| I (3) | 100 | 100 | 100 | 100 | 100 | 100 | 100 |
| II (4) | 80 | | 62 | 100 | 100 | | |
| III (5) | | | 90 | 100 | | | |
| IV (6) | 100 | 100 | 100 | 100 | 100 | 70 | 100 |
| VI (8) | 100 | 100 | 100 | 100 | 100 | | 100 |
| VII (9) | 100 | 100 | 100 | 86 | 100 | 53 | |
| VIII (10) | 100 | | 100 | 100 | 100 | 40 | 100 |

In the following Table 2 there has been reported the biological activity against acari eggs (Tetranichus urticae) of Compound No. 4 (example 19) in comparison with that of known pyrethroids.

TABLE 2

| Compound | Dose (%o) 0.1 | 0.05 |
|---|---|---|
| IV | 100 | 100 |
| Permethrin (a) | 18 | 0 |
| Cipermethrin (b) | 11 | 0 |
| Decamethrin (c) | 0 | 0 |
| Phenvalerate (d) | 18 | 0 |

(a) Permethrin ; 3-phenoxy-benzyl ester of 2,2-dimethyl-3-(β,β-dichlorovinyl)-cyclopropanecarboxylic acid ;

(b) Cipermethrin : α-cyano-3-phenoxy-benzyl ester of 2,2-dimethyl-3-(β,β-dichlorovinyl)-cyclopropanecarboxylic acid ;

(c) Decamethrin : α-cyano-3-phenoxy-benzyl ester of 2,2-dimethyl-3-(β,β-dibromovinyl)-cyclopropanecarboxylic acid ;

(d) Phenvalerate : α-cyano-3-phenoxy-benzyl ester of 1-(4′-chlorophenyl)-2-methyl-butyric acid.

## Claims

1. Compounds of general formula (I):

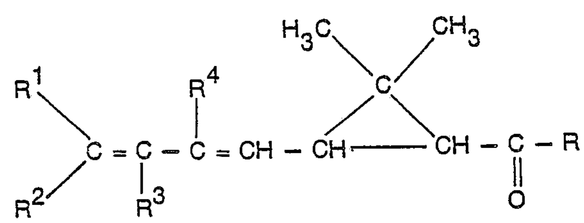

(I)

wherein R = OH, O-alkyl $C_1$—$C_4$, halogen,

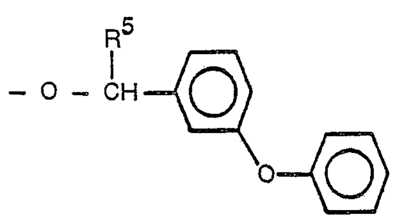

($R^5$ = H, CN, —C≡CH), $R^1$ = F, Cl, Br, $CH_3$, $CF_3$, $R^2$ = F, Cl, Br, $CF_3$, $R^3$ = H, F, Cl, Br, $CF_3$, $R^4$ = H, F, Cl, Br, $CF_3$, or $R^2$ and $R^3$ together form a third bond between the carbon atoms to which they are bonded.

2. Compounds of general formula I, according to claim 1, wherein:

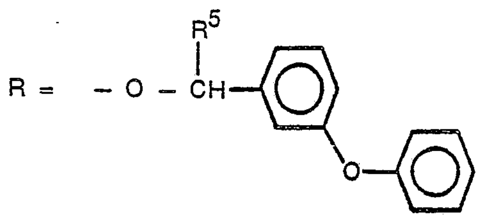

and $R^5$ = H, CN.

3. The α - cyano - 3 - phenoxy - benzyl ester of 3 - (4',4' - dichloro - butadienyl) - 2,2 - dimethyl - cyclopropanecarboxylic acid, of 3 -(4',4' - dichloro - 2' - bromo - butadienyl) - 2,2 - dimethyl - cyclopropanecarboxylic acid or of 3 - (2',4',4' - trichloro - butadienyl) - 2,2 - dimethyl - cyclopropanecarboxylic acid.

4. The 3 - phenoxy - benzyl ester of 3 - (4',4' - dichlorobutadienyl) - 2,2 - dimethyl - cyclopropanecarboxylic acid, of 3 - (3',4',4' - trichlorobutadienyl) - 2,2 - dimethyl - cyclopropanecarboxylic acid or of 3 - (4',4' - dichloro - 2' - bromo - butadienyl) - 2,2 - dimethyl - cyclopropanecarboxylic acid.

5. The α - cyano - 3 - phenoxy - benzyl ester of 3 - (5',5',5' - trifluoro - 4' - chloro-penta - 1',3' - dienyl) - 2,2 - dimethyl - cyclopropanecarboxylic acid or of 3 - (4' - chloro - 1',3' - pentadienyl) - 2,2 - dimethyl - cyclopropanecarboxylic acid.

6. Compounds of general formula I, according to claim 1, wherein: R = OH, O-alkyl $C_1$—$C_4$, halogen.

7. Compounds according to claim 6, having the formula:

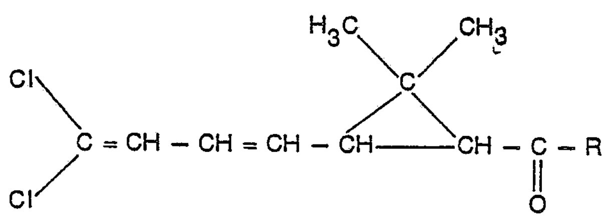

or

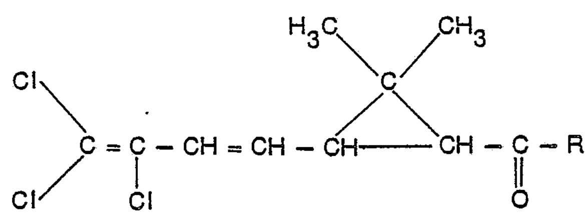

or

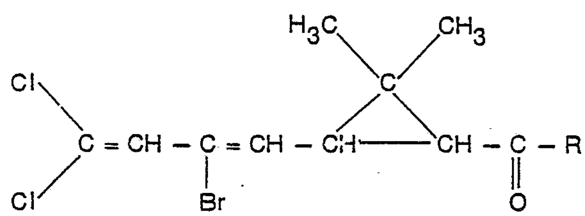

or

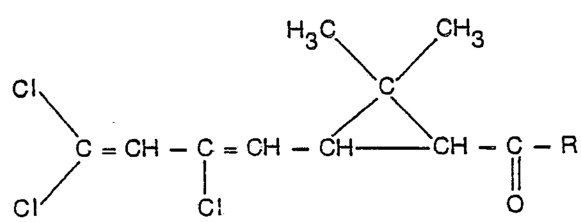

wherein : R = OH, $OC_2H_5$, Cl.

8. Compounds according to claim 6, having the formula:

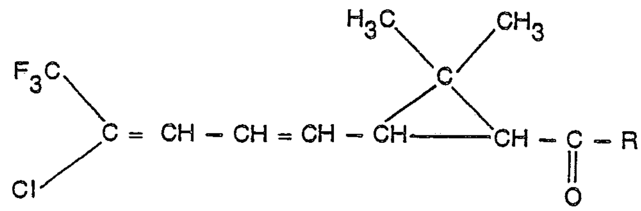

or

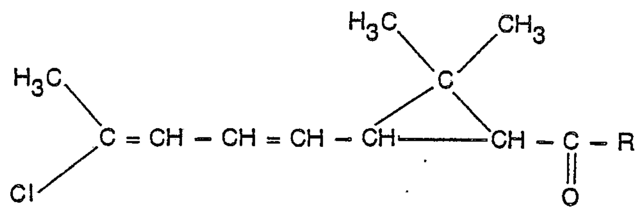

wherein : R = OH, $OC_2H_5$, Cl.

9. Process for preparing the compounds of claim 1, wherein $R^4$ is different from hydrogen, characterized in that a compound of the formula:

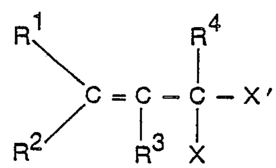

(3)

[wherein $R^1$, $R^2$ and $R^3$ have the meanings reported in claim 1; $R^4$ = F, Cl, Br, $CF_3$; X and X', either equal to or different from each other, = Cl, Br], is made to react with triphenyl-phosphine $(C_6H_5)_3P$ according to the equation:

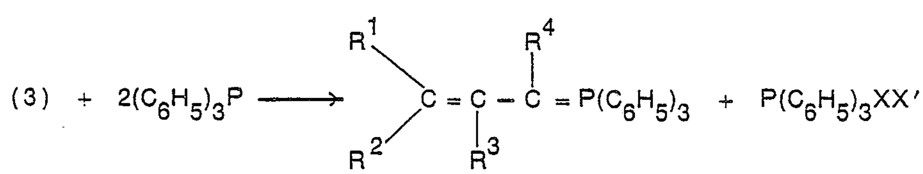

(2)

and the ylide (2) thus obtained is made to react with a lower alkyl ester of 2,2-dimethyl-3-formylcyclopropanecarboxylic acid (caronic aldehyde) thereby obtaining the compounds of general formula I, wherein $R^4 \neq H$ and R = O-alkyl, and from which, by hydrolysis, there are prepared the compounds of formula I wherein $R^4 \neq H$ and R = OH, which in their turn are treated with thionyl chloride ($SOCl_2$) thereby obtaining the compounds of formula I wherein $R^4 \neq H$ and R = Cl, which finally, by reaction with an alcohol of the formula:

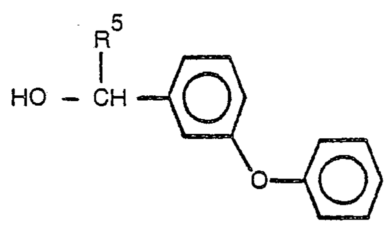

(II)

wherein $R^5$ = H, CN; —C≡CH,
give the compounds of formula I wherein $R^4 \neq H$ and where

R = 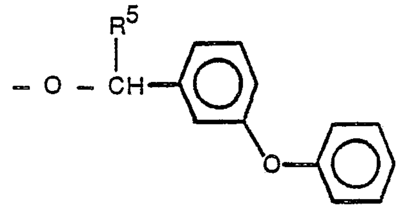

($R^5$ = H, CN, —C≡CH).

10. Process for preparing the compounds of claim 1, wherein $R^3$ = H, characterized in that an ester of 3,3-dimethyl-4-pentenoic or 2-alcoxycarbonyl-3,3-dimethyl-4-pentenoic acid, having the formula:

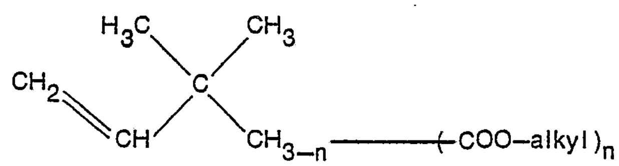

(5)

(wherein n = 1 or 2), is made to react, in the presence of radical reaction promoters, with a polyhaloalkane (propane or buthane) of the formula:

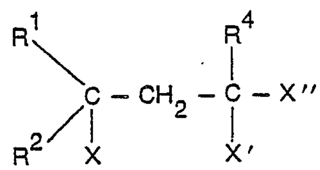

(4)

wherein $R^1$, $R^2$ and $R^4$ have the meanings indicated in claim 1, X, X′ and X″ = Cl or Br, thereby obtaining the compounds of formula:

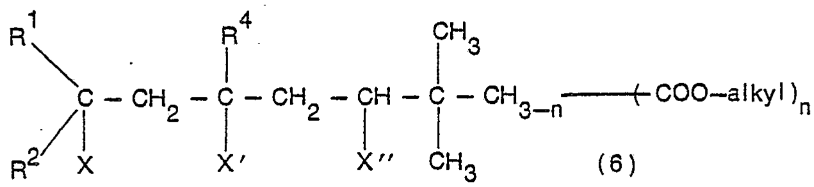

(6)

(wherein $R^1$, $R^2$, $R^3$, X, X′, X″ and *n* have the meanings previously reported), which by treatment with bases undergo in one or more stages, three successive dehydrohalogenations optionally followed (in the case n = 2) by a decarboxylation, affording the compounds of claim 1, wherein $R^3$ = H, R = OH, O-alkyl, which are converted to the corresponding acyl-chlorides (form. I, $R^3$ = H, R = Cl) and finally, from these, by reaction with an alcohol of the formula:

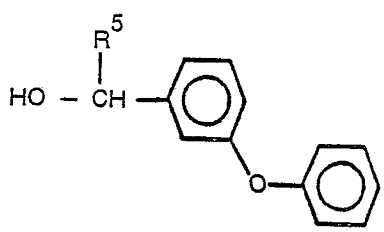

(II)

(wherein $R^5$ = H, CN; —C≡CH) there are obtained the compounds of claim 1 wherein $R^3$ = H and

R = 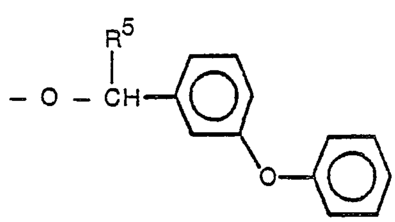

(wherein $R^5$ = H, CN, —C≡CH).

11. Process for the preparation of the compounds of claim 1, wherein $R^4 = H$, characterized in that a lower alkyl ester of 2,2-dimethyl-3-bromomethyl-cyclopropanecarboxylic acid of the formula:

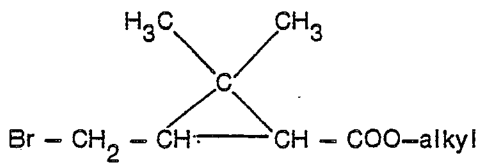

(11)

is converted by treatment with triphenylphosphine $(C_6H_5)_3P$, to the phosphonium salt of formula:

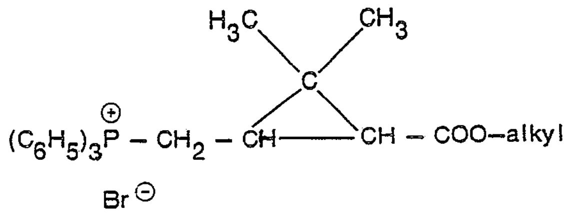

(13)

which, by reaction, in the presence of a strong base, with an aldehyde of the formula:

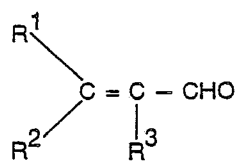

(14)

(wherein $R^1$, $R^2$ and $R^3$ have the meanings given to them in claim 1) affords the compounds of claim 1 of formula I wherein $R^4 = H$ and R = O-alkyl; said compounds, by hydrolysis yield the compounds of formula I wherein $R^4 = H$, R = OH, and which, by treatment with thionyl chloride ($SOCl_2$) afford the compounds of formula I wherein $R^4 = H$ and R = Cl, and, finally, from these latter, by reaction with an alcohol of the formula:

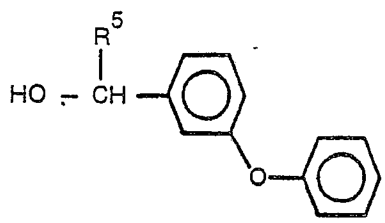

(II)

(wherein $R^5$ = H, CN, —C≡CH),
there are obtained the compounds of formula I wherein $R^4 = H$ and

R = 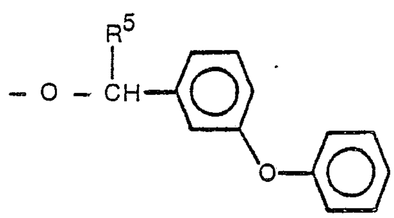

(wherein $R^5$ = H, CN, —C≡CH).

12. Process for the preparation of the compounds of claim 1, in which $R^3 = H$, $R^4$ = H, F, Cl, Br, characterized in that a phosphonate of formula:

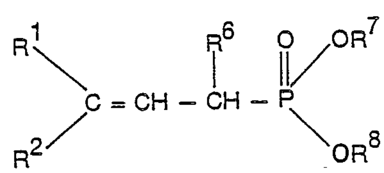

(VII)

wherein $R^1$ and $R^2$ have the meanings reported in claim 1, $R^6$ = H, F, Cl, Br and $R^7$ and $R^8$, equal to or different from each other, represent an alkyl or a haloalkyl having from 1 to 4 carbon atoms, or $R^7$ and $R^8$ together form either a

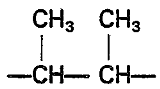 or a 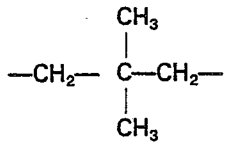 group;

is made to react at a temperature of between −50° and 100°C, in the presence of a strong base and in a suitable solvent or diluent, with a lower alkyl ester of 2,2-dimethyl-3-formyl-cyclopropanecarboxylic acid (caronic aldehyde) of following formula:

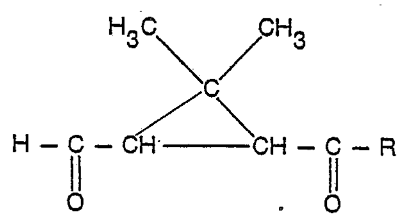

(wherein R = O–alkyl $C_1 - C_4$)

thereby obtaining the compounds of claim 1 in which $R^3$ = H, $R^4$ = H, F, Cl, Br and R = O-alkyl $C_1$—$C_4$ which are then converted into the corresponding carboxylic acid (R = OH) and acyl-chloride (R = Cl) which, in their turn, are made to react with an alcohol of formula:

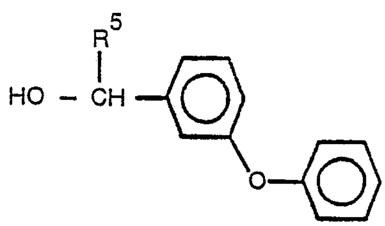

thus obtaining the compound of claim 1 in which:

R = 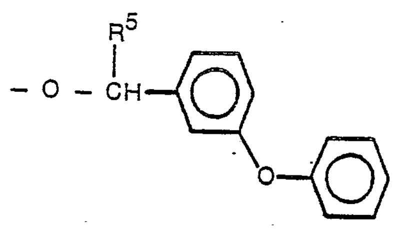

13. Process according to claim 12 characterized in that the reaction between phosphonate VII and the caronic aldehyde is conducted at a temperature comprised between −30°C and room temperature and/or

b) the strong base is either a hydride or a hydroxide of an alkaline metal and/or

c) as an inert solvent or diluent there is used an ether, an aprotic polar solvent or a mixture of polar-apolar solvents and/or

d) starting from lower alkyl esters of caronic aldehyde in the cis or trans form, there are obtained the cyclopropanecarboxylic esters in the corresponding isomeric form and/or

e) the reaction between phosphonate VII and the alkyl ester of the caronic aldehyde, is carried out by admixing a mixture of the aforesaid compounds to a suspension or solution of the strong base in a suitable solvent or

f) the reaction between the phosphonate VII and the alkyl ester of the caronic aldehyde is carried out by adding a solution of phosphonate VII in a suitable solvent to a suspension or solution of the base and of the alkyl ester of caronic aldehyde in a suitable solvent.

14. Insecticide and/or acaricide compositions having as an active principle one or more of the compounds of claim 1, wherein

R = 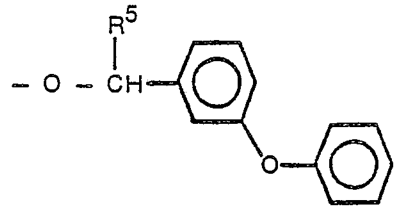

**Patentansprüche**

1. Verbindungen der allgemeinen Formel (I)

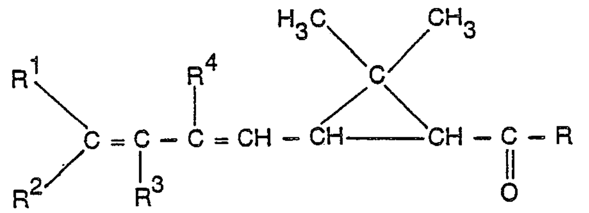

(I)

in welcher

R = OH, O—Alkyl $C_1$—$C_4$, Halogen, 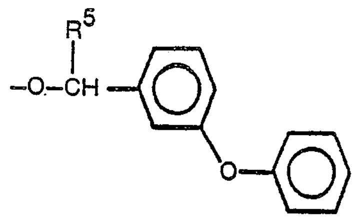

($R^5$ = H, CN, —C≡CH), $R^1$ = F, Cl, Br, $CH_3$, $CF_3$, $R^2$ = F, Cl, Br, $CF_3$, $R^3$ = H, F, Cl, Br, $CF_3$, $R^4$ = H, F, Cl, Br, $CF_3$, sind.

oder $R^2$ und $R^3$ bilden zusammen eine dritte Bindung zwischen den Kohlenstoffatomen, an welche sie gebunden sind.

2. Verbindungen der allgemeinen Formel (I) gemäß Anspruch 1, in welchen

R = 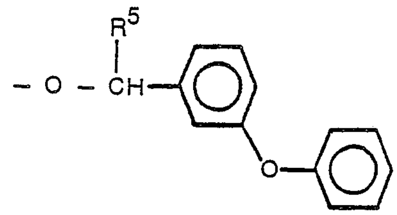

und $R^5$ = H, CN sind.

3. Der α - Cyan - 3 - phenoxybenzylester der 3 - (4′,4′ - Dichlorbutadienyl) - 2,2 - dimethyl - cyclopropancarbonsäure, der 3 - (4′,4′ - Dichlor - 2′ - brombutadienyl) - 2,2 - dimethylcyclopropancarbonsäure oder der 3 - (2′,4′,4′ - trichlorbutadienyl) - 2,2 - dimethylcyclopropancarbonsäure.

4. Der 3 - Phenoxybenzylester der 3 - (4′,4′ - Dichlorbutadienyl) - 2,2 - dimethylcyclopropancarbonsäure, der 3 - (3′,4′,4′ - trichlorbutadienyl) - 2,2 - dimethylcyclopropancarbonsäure oder der 3 - (4′,4′ - Dichlor - 2′ - brombutadienyl) - 2,2 - dimethylcyclopropancarbonsäure.

5. Der α - Cyan - 3 - phenoxybenzylester der 3 - (5′,5′,5′ - Trifluor - 4′ - chlorpenta - 1′,3′ - dienyl) - 2,2 - dimethylcyclopropancarbonsäure oder der 3 - (4′ - Chlor - 1′,3′ - pentadienyl) - 2,2 - dimethylcyclopropancarbonsäure.

6. Verbindungen der allgemeinen Formel (I) gemäß Anspruch 1, in welchen R = OH, O-Alkyl $C_1$—$C_4$, Halogen ist.

7. Verbindungen nach Anspruch 6 mit der Formel

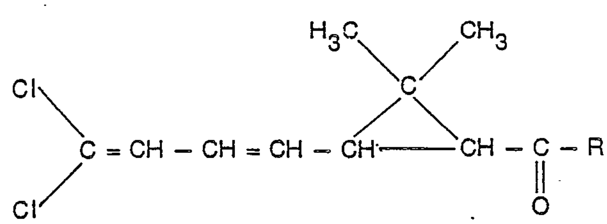

oder

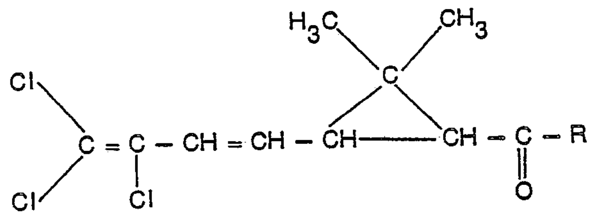

oder

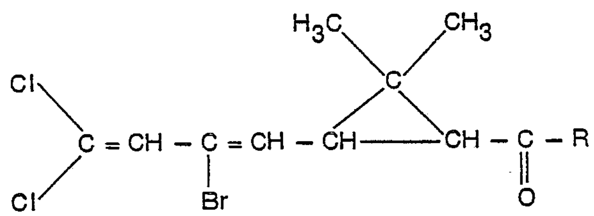

oder

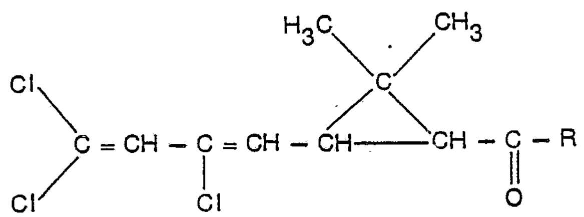

in welcher R = OH, $OC_2H_5$, Cl ist.

8. Verbindungen nach Anspruch 6 mit der Formel:

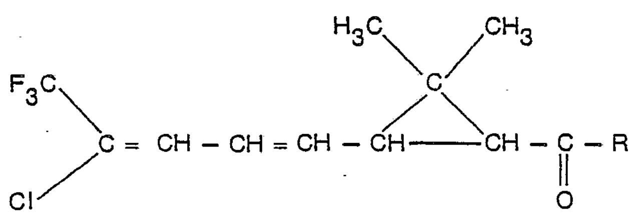

oder

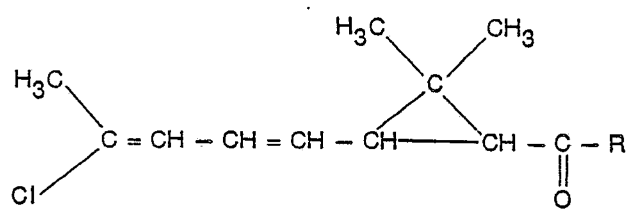

in welcher R = OH, $OC_2H_5$, Cl ist.

9. Verfahren zur Herstellung der Verbindungen von Anspruch 1, in welchen $R^4$ von Wasserstoff verschieden ist, dadurch gekennzeichnet, daß man eine Verbindung der Formel:

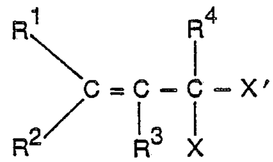

(3)

[in welcher $R^1$, $R^2$ und $R^3$ die in Anspruch 1 angegebene Bedeutung haben; $R^4$ = F, Cl, Br, $CF_3$; X und X′ entweder miteinander gleich oder voneinander verschieden sind und = Cl, Br], mit Triphenylphosphin $(C_6H_5)_3P$ gemäß der Gleichung

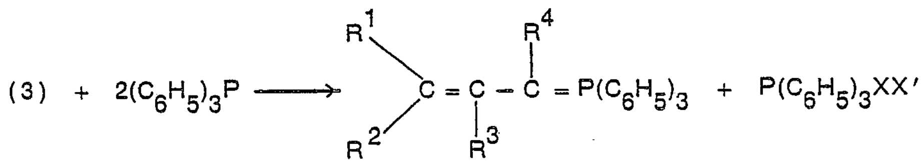

(2)

reagieren läßt und das so erhaltene Ylid (2) mit einem niedrigen Alkylester der 2,2-Dimethyl-3-formylcyclopropancarbonsäure (Caronsäurealdehyd) zur Herstellung der Verbindungen der allgemeinen Formel (I) umsetzt, in welchen $R^4 \neq H$ und R = O-Alkyl, aus welchen durch Hydrolyse Verbindungen der Formel (I) hergestellt werden, in welchen $R^4 \neq H$ und R = OH sind, die ihrerseits mit Thionylchlorid ($SOCl_2$) zur Herstellung der Verbindungen der Formel (I) behandelt werden, in welchen $R^4 \neq H$ und R = Cl sind, die schließlich durch Reaktion mit einem Alkohol der Formel:

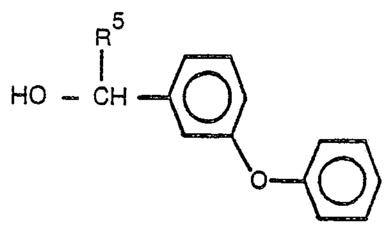

(II)

in welcher $R^5$ = H, CN, —C≡CH ist, Verbindungen der Formel (I) liefern, in welchen $R^4 \neq H$ und

R =

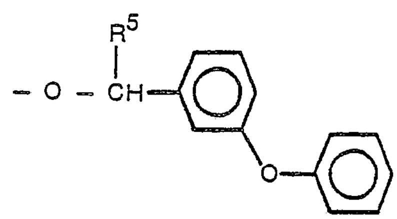

($R^5$ = H, CN, —C≡CH) sind.

10. Verfahren zur Herstellung der Verbindungen von Anspruch 1, in welchen $R^3$ = H, dadurch gekennzeichnet, daß man einen Ester der 3,3-Dimethyl-4-penten- oder 2-Alkoxycarbonyl-3,3-dimethyl-4-pentensäure mit der Formel:

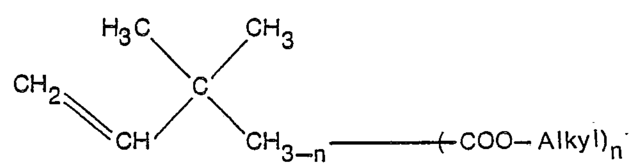

(5)

(in welcher n = 1 oder 2) in Anwesenheit eines Radikalreaktionsbeschleunigers mit einem Polyhalogenalkan (Propan oder Butan) der Formel:

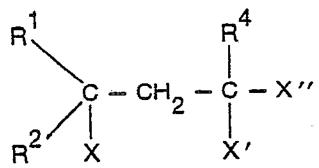

(4)

in welcher $R^1$, $R^2$ und $R^4$ die in Anspruch 1 angegebene Bedeutung haben und X, X′ und X″ = Cl oder Br sind, umsetzt, wodurch man die Verbindungen der Formel

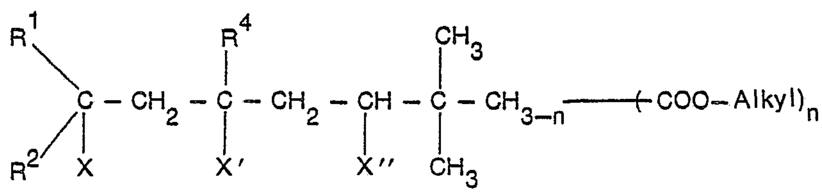

(6)

(in welcher $R^1$, $R^2$, $R^3$, X, X′ und X″ sowie n die oben angegebene Bedeutung haben) erhält, die durch Behandlung mit Basen in einer oder mehreren Stufen drei aufeinander folgenden Dehydrohalogenierungen unterliegen, die wahlweise (falls n = 2) durch eine Decarboxylierung gefolgt werden, wodurch man die Verbindungen von Anspruch 1 erhält, in welchen $R^3$ = H, und R = OH, O-Alkyl sind, die in die entsprechenden Acylchloride (Formel (I), $R^3$ = H, R = Cl) umgewandelt werden, und aus denen man schließlich durch Reaktion mit einem Alkohol der Formel:

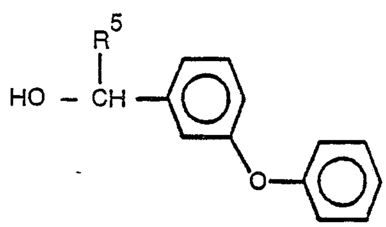

(in welcher $R^5$ = H, CN, —C≡CH) die Verbindungen von Anspruch 1 erhält, in welchen $R^3$ = H und

R = 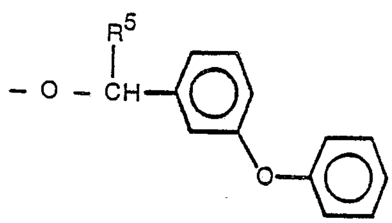

($R^5$ = H, CN, —C≡CH) sind.

11. Verfahren zur Herstellung der Verbindungen von Anspruch 1, in welchen $R^4$ = H, dadurch gekennzeichnet, daß man einen niedrigen Alkylester der 2,2-Dimethyl-3-brommethyl-cyclopropancarbonsäure der Formel:

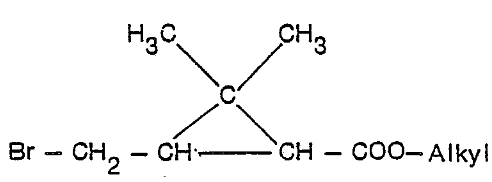

(11)

durch Behandlung mit Triphenylphosphin $(C_6H_5)_3P$ in das Phosphoniumsalz der Formel:

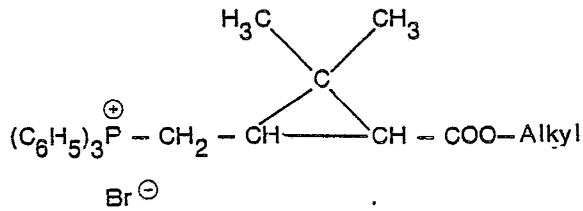

(13)

umwandelt, das durch Reaktion in Anwesenheit einer starken Base mit einem Aldehyd der Formel:

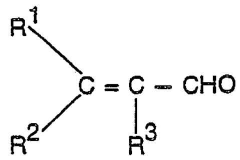

(14)

(in welcher $R^1$, $R^2$ und $R^3$ die in Anspruch 1 angegebene Bedeutung haben) die Verbindungen der Formel (I) von Anspruch 1 liefert, in welchen $R^4 = H$ und R = O-Alkyl sind, wobei diese Verbindungen durch Hydrolyse die Verbindungen der Formel (I) liefern, in welchen $R^4 = H$ und R = OH sind, und die durch Behandlung mit Thionylchlorid ($SOCl_2$) die Verbindungen der Formel (I) liefern, in welcher $R^4 = H$ und R = Cl sind, worauf man schließlich aus den letzteren durch Reaktion mit einem Alkohol der Formel:

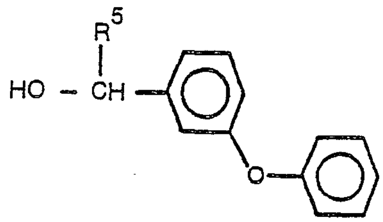

($R^5$ = H, CN, —C≡CH) die Verbindungen der Formel (I) erhält, in welchen $R^4 = H$ und

R =

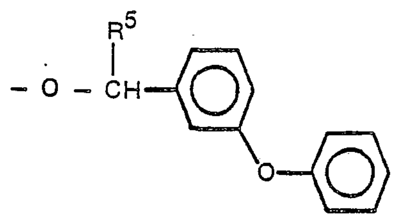

($R^5$ = H, CN, —C≡CH) sind.

12. Verfahren zur Herstellung der Verbindungen von Anspruch 1, in welchen $R^3 = H$, $R^4$ = H, F, Cl, Br, dadurch gekennzeichnet, daß man ein Phosphonat der Formel:

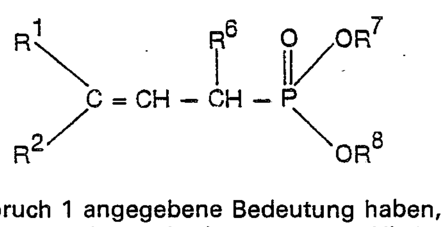

(VII)

in welcher $R^1$ und $R^2$ die in Anspruch 1 angegebene Bedeutung haben, $R^6$ = H, Cl, Br ist und $R^7$ und $R^8$, die gleich oder voneinander verschieden sein können, ein Alkyl oder Halogenalkyl mit 1 bis 4 Kohlenstoffatom darstellen, oder $R^7$ und $R^8$ bilden zusammen entweder eine

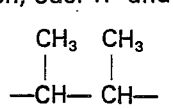 oder eine 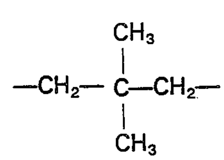 Gruppe,

bei einer Temperatur zwischen −50° und 100°C in Anwesenheit einer starken Base und in einem geeigneten Lösungs- oder Verdünnungsmittel mit einem niedrigen Alkylester der 2,2-Dimethyl-3-formyl-cyclopropancarbonsäure (Caronsäurealdehyd) der folgenden Formel:

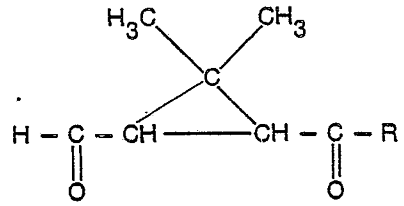

(R = O-Alkyl $C_{1-4}$) umsetzt, wodurch man die Verbindungen von Anspruch 1 erhält, in welchen $R^3$ = H, $R^4$ = H, F, Cl, Br und R = O-Alkyl $C_{1-4}$ sind, die dann in die entsprechende Carbonsäure (R = OH) und das Acylchlorid (R = Cl) umgewandelt werden, die ihrerseits mit einem Alkohol der Formel:

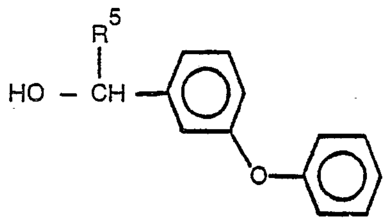

(II)

zur Bildung der Verbindungen von Anspruch 1 umgesetzt werden, in welchen

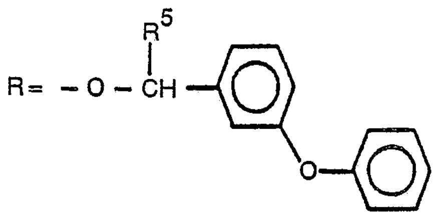

ist.

13. Verfahren nach Anspruch 12, dadurch gekennzeichnet, daß die Reaktion zwischen dem Phosphonat (VII) und dem Caronsäurealdehyd bei einer Temperatur zwischen −30°C und Raumtemperatur durchgeführt wird, und/oder

b) die starke Base entweder ein Hydrid oder ein Hydroxid eines Alkalimetalls ist, und/oder

c) als inertes Lösungs- oder Verdünnungsmittel ein Ether, ein aprotisches polares Lösungsmittel oder eine Mischung polar/apolarer Lösungsmittel verwendet wird, und/oder

d) man, ausgehend von niedrigen Alkylestern des Caronsäurealdehyds in der cis- oder trans-form, die Cyclopropancarbonsäureester in der entsprechenden isomeren Form erhält und/oder

e) die Reaktion zwischen dem Phosphonat (VII) und dem Alkylester des Caronsäurealdehyds durchgeführt wird, indem man eine Mischung der genannten Verbindungen zu einer Suspension oder Lösung der starken Base in einem geeigneten Lösungsmittel einmischt, oder

f) man die Reaktion zwischen dem Phosphonat (VII) und dem Alkylester des Caronsäurealdehyds durchführt, indem man eine Lösung des Phosphonates (VII) in einem geeigneten Lösungsmittel zu einer Suspension oder Lösung aus der Base und dem Alkylester des Caronsäurealdehyds in einem geeigneten Lösungsmittel zufügt.

14. Insektizide und/oder acarizide Zusammensetzungen, die als aktiven Bestandteil eine oder mehrere Verbindungen von Anspruch 1 enthalten, in welchen

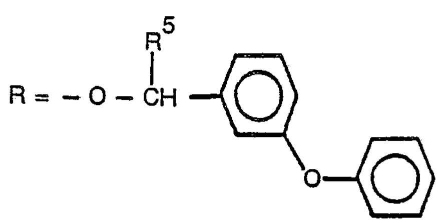

ist.

**Revendications**

1. Composés de formule générale (I):

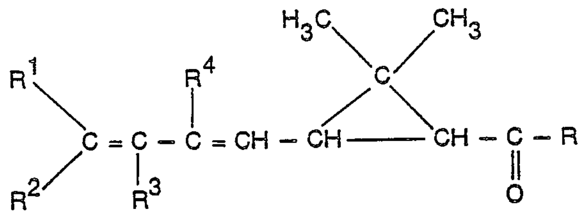

(I)

dans laquelle: R = OH, O-alkyle comprenant 1 à 4 atomes de carbone, halogène,

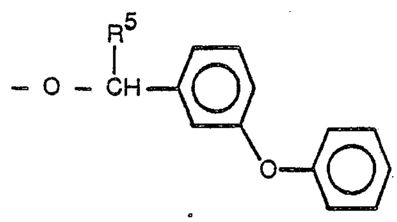

($R^5$ = H, CN, —C≡CH), $R^1$ = F, Cl, Br, $CH_3$, $CF_3$; $R^2$ = F, Cl, Br, $CF_3$; $R^3$ = H, F, Cl, Br, $CF_3$; $R^4$ = H, F, Cl, Br, $CF_3$; ou $R^2$ et $R^3$ forment ensemble une troisième liaison entre les atomes de carbone auxquels ils sont liés.

2. Composés de formule générale I, selon la revendication 1, dans laquelle:

R =

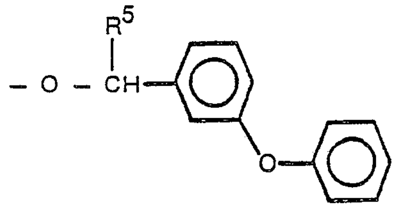

et $R^5$ = H, CN.

3. L'ester alpha - cyano - 3 - phénoxy - benzylique de l'acide 3 - (de l'acide 3 - (4',4' - dichloro - 2' - bromo butaiényl) - 2,2 - diméthyl - cyclopropanecarboxylique ou de l'acide 3 - (2',4',4' - trichloro - butadiényl) - 2,2 - diméthylcyclopropanecarboxylic.

4. L'ester 3 - phénoxy-benzylique de l'acide 3 - (4',4' - dichlorobutadiényl) - 2,2 - diméthyl - cyclopropanecarboxylique, de l'acide 3 - (3',4',4' - trichlorobutadiényl) - 2,2 - diméthyl - cyclopropanecar boxylique ou de l'acide 3 - (4',4' - dichloro - 2' - bromo - butadiényl) - 2,2 - diméthyl - cyclopropanecarboxy lique.

5. L'ester alpha - cyano - 3 - phénoxy - benzylique de l'acide 3 - (5',5',5' - trifluoro - 4' - chloro - penta - 1',3' - diényl) - 2,2 - diméthyl - cyclopropanecarboxylique ou de l'acide 3 - (4' - chloro - 1',3' - pentadiényl) - 2,2 - diméthyl - cyclopropanecarboxylique.

6. Composés de formule générale I, selon la revendication 1, dans laquelle: R = OH, O-alkyle comprenant 1 à 4 atomes de carbone, halogène.

7. Composés selon la revendication 6, présentant la formule:

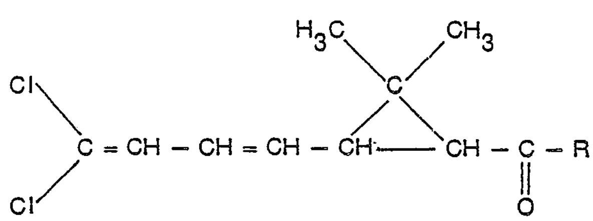

ou

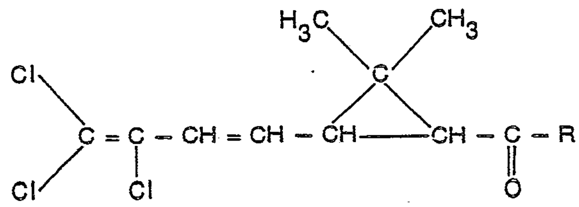

ou

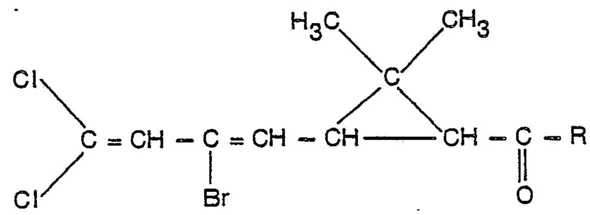

ou

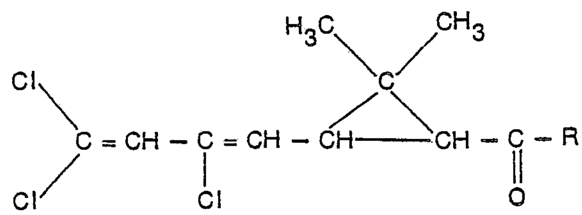

dans lesquelles: R = OH, $OC_2H_5$, Cl.

8. Composés selon la revendication 6, présentant la formule:

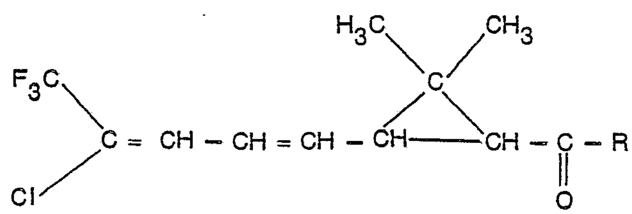

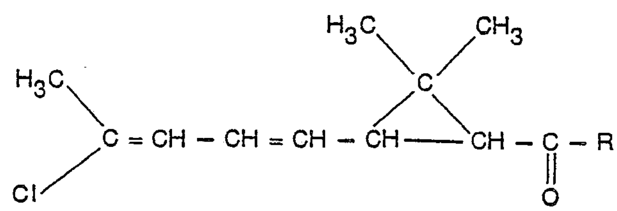

dans lesquelles: R = OH, $OC_2H_5$, Cl.

9. Procédé de préparation des composés selon la revendication 1, dans lequel $R^4$ est autre que de l'hydrogène, caractérisé en ce qu'un composé de formule:

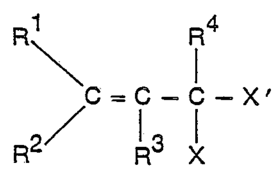 (3)

dans laquelle: $R^1$, $R^2$, $R^3$ ont les significations mentionnées dans la revendication 1; $R^4$ = F, Cl, Br, $CF_3$; X et X′, identiques ou différents l'un de l'autre, = Cl, Br, sont amenés avec une triphénylphosphine $(C_6H_5)_3P$ selon l'équation:

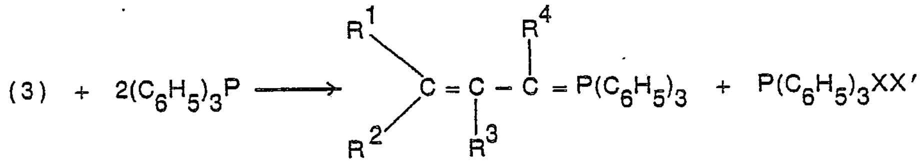

(2)

et l'ylure (2) ainsi obtenu est amené à réagir avec un ester d'alkyle inférieur de l'acide 2,2-diméthyl-3-formyl-cyclopropanecarboxylique (aldéhyde caronique) de façon à conduire aux composés de formule générale I dans laquelle $R^4$ est différent de H et R = O-alkyle, et à partir duquel, par hydrolyse, sont préparés les composés de formule I dans laquelle $R^4$ est différent de H et R = OH qui, à leur tour, sont traités par le chlorure de thionyle ($SOCl_2$) de façon à conduire aux composés de formule I dans laquelle $R^4$ est différent de H et R = Cl qui, enfin, par réaction avec un alcool de formule:

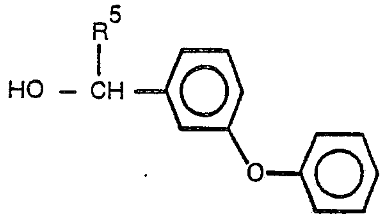

(II)

dans laquelle: $R^5$ = H, CN; —C≡CH, conduit aux composés de formule I dans laquelle $R^4$ est différent de H et où:

R = 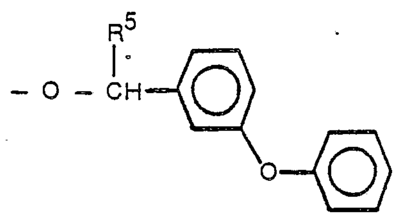

($R^5$ = H, CN, —C≡CH).

10. Procédé de préparation des composés selon la revendication 1, avec $R^3$ = H, caractérisé en ce qu'un ester de l'acide 3,3-diméthyl-4-penténoïque ou de l'acide 2-alcoxycarbonyl-3,3-diméthyl-4-penténoïque, présentant la formule:

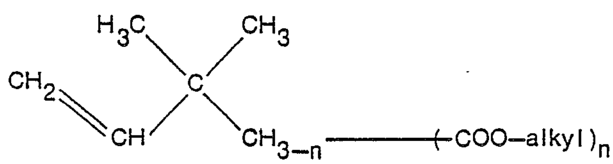

(5)

(dans laquelle n = 1 ou 2), est amené à réagir en présence de promoteurs de réaction radicalaire avec un polyhaloalkane (propane ou butane) de formule:

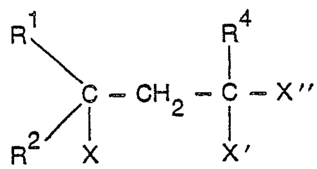

(4)

dans laquelle: $R^1$, $R^2$, $R^4$ ont les significations indiquées dans la revendication 1, X, X′ ou X″ = Cl ou Br, de façon à conduire aux composés de formule:

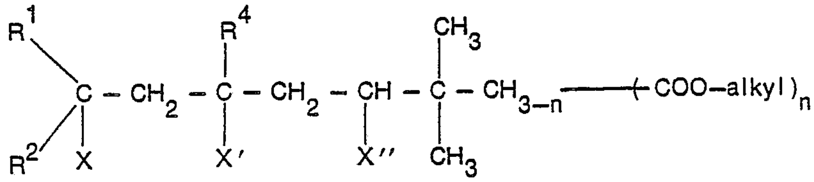

(6)

(dans laquelle $R^1$, $R^2$, $R^3$, X, X', X'' et n ont les significations précitées), qui, par traitement avec des bases, subissent en une ou plusieurs étapes trois déshydrohalogénations successives éventuellement suivies (dans le cas où n = 2) une décarboxylation, ce qui conduit aux composés selon la revendication 1 avec $R^3$ = H, R = OH, O-alkyle, qui sont convertis en les chlorures d'acyle correspondants (form. I, $R^3$ = H, R = Cl) et enfin, à partir de ceci par réaction avec un alcool de formule:

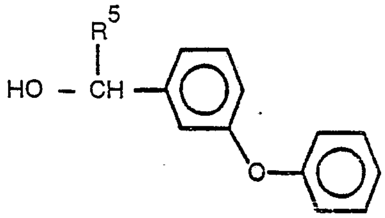

(dans laquelle $R^5$ = H, CN; —C≡CH), on obtient les composés selon la revendication 1 avec $R^3$ = H et

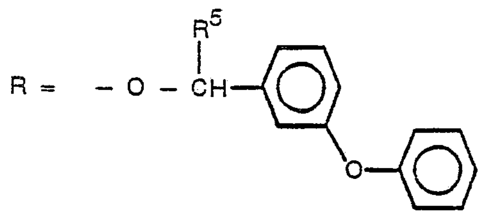

(dans laquelle $R^5$ = H, CN; —C≡CH).

11. Procédé de préparation des composés selon la revendication 1, avec $R^4$ = H, caractérisé en ce qu'un ester d'alkyle inférieur de l'acide 2,2-diméthyl-3-bromométhyl-cyclopropanecarboxylique de formule:

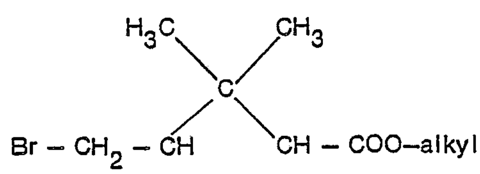

(11)

est converti par traitement avec une triphénylphosphine $(C_6H_5)_3P$, en le sel phosphonium de formule:

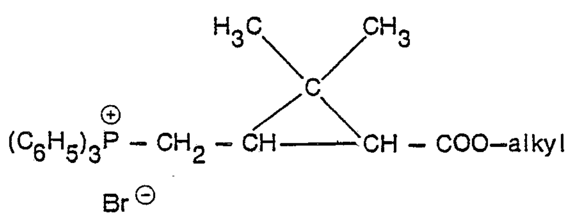

(13)

qui, par réaction en présence d'une base forte avec un aldéhyde de formule:

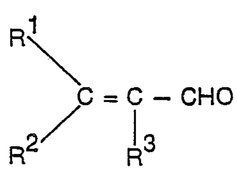

(14)

(dans laquelle $R^1$, $R^2$, $R^3$ ont les significations indiquées dans la revendication 1), ce qui conduit aux composés selon la revendication de formule I dans laquelle $R^4$ = H et R = O-alkyle, lesdits composés, par hydrolyse, conduisant aux composés de formule I dans $R^4$ = H et R = OH et qui, par traitement avec du chlorure de thionyle ($SoCl_2$) conduisent aux composés de formule I dans laquelle $R^4$ = H et R = Cl et, enfin, à partir de ces derniers par réaction avec un alcool de formule:

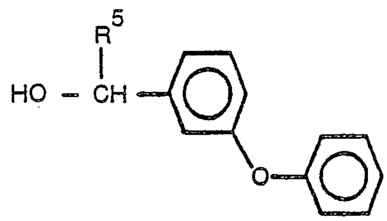

(II)

(dans laquelle $R^5$ = H, CN, —C=CH), on obtient les composés de formule I dans laquelle $R^4$ = H et

R = 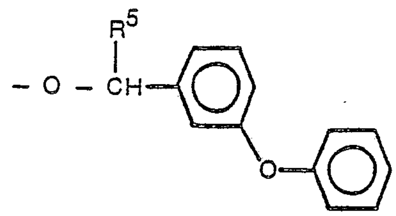

(dans laquelle $R^5$ = H, CN, —C≡CH).

12. Procédé de préparation des composés selon la revendication 1, avec $R^3$ = H et $R^4$ = H, F, Cl, Br, caractérisé en ce qu'un phosphonate de formule:

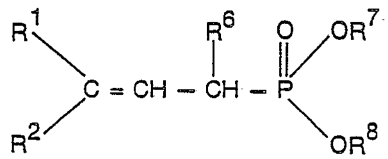

(VII)

dans laquelle: $R^1$, $R^2$, $R^3$ ont les significations indiquées dans la revendication 1, $R^6$ = H, F, Cl, Br et $R^7$ et $R^8$ indentiques ou différents les uns des autres représentent un radical alkyle ou haloalkyle comprenant 1 à 4 atomes de carbone, ou $R^7$ et $R^8$ forment ensemble soit un groupe

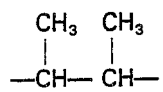 ou un groupe 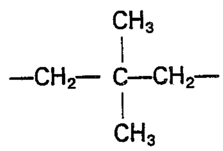

est amené à réagir à une température comprise entre −50° et 100°C, en présence d'une base forte et d'un solvant ou diluant convenable, avec un ester d'alkyle inférieur de l'acide 2,2-diméthyl-3-formyl-cyclopropane-carboxylique (aldéhyde caronique) ayant la formule suivante:

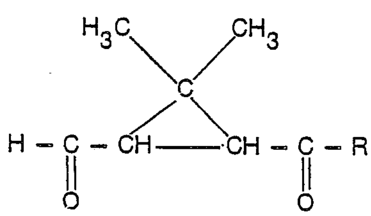

(dans laquelle R = O-alkyle comprenant 1 à 4 atomes de carbone), de façon à conduite aux composés selon la revendication 1, avec $R^3$ = H, $R^4$ = H, F, Cl, Br et R = O-alkyle comprenant 1 à 4 atomes de carbone, qui sont alors convertis en l'acide carboxylique correspondant (R = OH) et chlorure d'acyle (R = Cl) qui, à leur tour, sont amenés à réagir avec un alcool de formule:

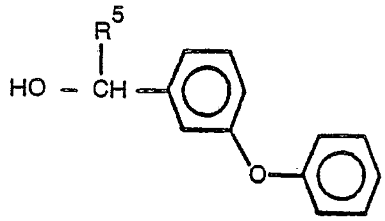

(II)

de façon à conduire aux composés selon la revendication 1 dans laquelle:

R = 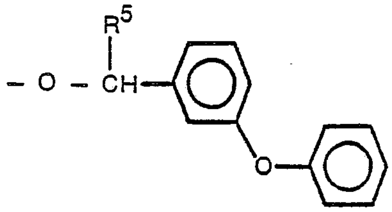

13. Procédé selon la revendication 12, caractérisé en ce que la réaction avec le phosphonate VII et l'aldéhyde caronique est effectuée à une température comprise entre −30°C et la température ambiante et/ou

b) la base forte et soit un hydrure soit un hydroxyde d'un métal alcalin et/ou

c) en tant que solvant ou diluant inerte, est utilisé un éther, un solvant polaire aprotique ou un mélange de solvants polaire et apolaire et/ou

d) en partant d'esters d'alkyle inférieur de l'aldéhyde caronique sous la forme cis ou trans, on obtient les esters cyclopropane-carboxyliques dans la forme isomère correspondante et/ou

e) réaction entre le phosphonate VII et l'ester alkylique de l'aldéhyde caronique est effectuée par addition d'un mélange des composés précités à une suspension ou solution de la base forte dans un solvant convenable ou

f) la réaction entre le phosphonate VII et l'ester alkylique de l'aldéhyde caronique est mise en oeuvre par addition d'une solution de phosphonate VII dans un solvant convenable à une suspension ou solution de la base et de l'ester alkylique de l'aldéhyde caronique dans un solvant convenable.

14. Composition insecticide et/ou acaricide présentant en tant que constituant principal un ou plusieurs des composés selon la revendication 1, où

R =